# EUROPÄISCHE PATENTANMELDUNG

(11) **EP 2 845 579 A2**
(43) Veröffentlichungstag der Anmeldung: **11.03.2015**
(21) Anmeldenummer: 14183659.3
(22) Anmeldetag: 05.09.2014
(51) Int. Cl.: A61K 6/00

(54) **Applikationssystem zur einfachen, 3-dimensionalen Anwendung von medizinischen, kosmetischen oder Arzneimittel enthaltenden Zahnpflegeprodukten**

(30) Priorität: 09.09.2013 DE 102013109848
(71) Anmelder: Heraeus Kulzer GmbH, 63450 Hanau (DE)
(72) Erfinder: Busch, Susanne, 61267 Neu Anspach (DE); Utterodt, Andreas, 61267 Neu Anspach (DE); Gerlach, Michael, 65719 Hofheim (DE); Suffel, Ralf, 61267 Neu Anspach (DE)
(74) Vertreter: Bendele, Tanja

(57) **Zusammenfassung**

Es wird ein teilelastischer Formkörper in Form eines Zahnbogennegativs enthaltend eine Matrix basierend auf einem Gel mit einem Wirkstoff oder einem mineralisierend wirkendem System-sowie ein Applikationssystem zur Zahnbehandlung offenbart. Das Applikationssystem umfasst eine Applikations-Zahnschiene, die zur Anwendung am Ober- und/oder Unterkiefer geeignet ist, wobei die Zahnschiene mindesten einen an die Schiene angepassten ersten dreidimensionalen Formkörper enthaltend eine Matrix aufnehmen kann, vorzugsweise ist die Zahnschiene thermisch verformbar. Die Matrix des mindestens einen ersten und optional weiteren Formkörpers enthält mindestens einen Wirkstoff oder ein mineralisierend wirkendes System mit Phosphaten, Calcium und Fluoriden. Ebenso Gegenstand der Erfindung ist das Verfahren zur Herstellung des Applikationssystems und ein Kit umfassend das Applikationssystem. Das Applikationssystem und Kit werden erfindungsgemäße zur Abscheidung von Apatit, insbesondere von nadelförmigen Fluorapatitkristalliten, angewendet. Erfindungsgemäss ist es möglich innerhalb einer Schlafperiode oder tagsüber grösser gleich 1 µm Apatit, insbesondere Fluorapatit auf Zahnoberflächen abzuscheiden, um poröse Zahnoberflächen zu verschliessen oder aufzuhellen.

## Beschreibung

Es wird ein teilelastischer borgenförmiger Formkörper in Form eines Zahnbogennegativs enthaltend eine Matrix basierend auf einem Gel mit einem Wirkstoff oder einem mineralisierend wirkendem System sowie ein Applikationssystem zur Zahnbehandlung offenbart. Das Applikationssystem umfasst eine Applikations-Zahnschiene, die zur Anwendung am Ober- und/oder Unterkiefer geeignet ist, wobei die Zahnschiene mindesten einen an die Schiene angepassten ersten dreidimensionalen Formkörper enthaltend eine Matrix aufnehmen kann, vorzugsweise ist die Zahnschiene thermisch verformbar. Die Matrix des mindestens einen ersten und optional weiteren Formkörpers enthält mindestens einen Wirkstoff oder ein mineralisierend wirkendes System mit Phosphaten, Calcium und Fluoriden. Ebenso Gegenstand der Erfindung ist das Verfahren zur Herstellung des Applikationssystems und ein Kit umfassend das Applikationssystem. Das Applikationssystem und Kit werden erfindungsgemäße zur Abscheidung von Apatit, insbesondere von nadelförmigen Fluorapatitkristalliten, angewendet. Erfindungsgemäss ist es möglich innerhalb einer Schlafperiode oder tagsüber grösser gleich 1 µm Apatit, insbesondere Fluorapatit auf Zahnoberflächen abzuscheiden, um poröse Zahnoberflächen zu verschliessen oder aufzuhellen.

Zähne sind harte Biomaterialien in Form von Verbundstoffen basierend auf Proteinen und Apatit umfassend Calcium und Phosphat. Der Zahnschmelz, die äussere Schicht der Zahnkrone ist der härteste Teil des Zahns und enthält keine lebenden Zellen. Er besteht aus anorganischen Kristallen, welche typische hoch orientierte Anordnungen aufweisen. Zahnschmelz ist ein Gewebe, das, sobald es einmal gebildet ist, lebenslang nahezu unverändert bleibt, da die Zellen, welche beim Aufbau der Zähne beteiligt sind, absterben, sobald die Zahnbildung abgeschlossen ist. Ein fertiger Zahnschmelz besteht aus etwa 95 Gew. -% Apatit, etwa 3 Gew. -% Proteinen und Lipiden und etwa 2 Gew. -% Wasser.

Um Schädigungen von Zähnen, insbesondere durch Karies, zu vermeiden oder zu reparieren, wurde seit langem versucht, remineralisierende Systeme einzusetzen. Dabei wurde zunächst versucht, durch Aufbringen von Calciumphosphat-verbindungen die Beschaffenheit der Zähne zu verbessern.

Solche Einkomponentensysteme, bei denen versucht wird, bereits vorgefertigtes Zahnmaterial, beispielsweise Apatit, Hydroxyapatit oder andere Calciumphosphat-verbindungen auf die Zähne aufzubringen, sind unter anderem in EP 0 666 730 B1 oder WO 01/95863 beschrieben. Das Problem solcher Systeme besteht darin, dass das Behandeln von Zahnmaterial mit Calciumphosphat-Verbindungen nicht zu einem Aufwachsen von strukturell dem Zahnmaterial ähnlichen Apatit führt, sondern vielmehr höchstens zu einer blossen Anlagerung von Apatitkristallen auf dem Zahnmaterial, wobei die Apatitkristalle eine vom Zahnmaterial völlig unterschiedliche Morphologie aufweisen. Somit wird keine Festigung des Zahnschmelzes oder dauerhafte Füllung von Läsionen bewirkt, da die angelagerten Apatitkristalle keine ausreichende Ähnlichkeit und Haftung zum natürlichen Zahnmaterial aufweisen.

Bedingt durch moderne Ernährungsgewohnheiten die häufig saure Lebensmittel enthalten, nehmen nicht bakteriell bedingte Erosionen an der Zahnhartsubstanz zu [Dentale Erosionen: Von der Diagnose zur Therapie, Adrian Lussi, Thomas Jaeggi, Quitessenz Verlag].

Nicht nur Lebensmittel wie stark gesäuerte Süssigkeiten, Softdrinks oder Alkopops spielen hier eine Rolle, sondern auch eine zunehmend stark obsthaltige Ernährung kann zu Zahnproblemen führen. Durch die kontinuierliche Einwirkung von Säuren wird der Zahnschmelz dünner und auch poröser. In Extremfällen kann der Schmelz vollständig aufgelöst bzw. abradiert werden, das empfindliche Dentin liegt dann frei. Insbesondere im Zahnhalsbereich, der nur von einer sehr dünnen Schicht Schmelz geschützt wird, tritt dies häufig auf. Die säurebedingte Erosion kann dann noch schneller weiter voranschreiten, da Dentin säurelöslicher ist als Schmelz und es kommt häufig zu keilförmigen Defekten der Zahnhartsubstanz. Freiliegendes Dentin führt zu sensiblen, schmerzempfindlichen Zähnen. Sensible Zahnhälse können aber auch die Folge falscher Putzgewohnheiten sein. Auch zunehmendes Alter ist ein Grund für dünner werdenden Schmelz. Regelmässiges Knirschen kann den Schmelz an den Schneidekanten abradieren. Da durch verbesserte Prophylaxe in der Zahnheilkunde und die konsequente Fluoridierung der meisten Zahncremes und zusätzlicher Pflegeprodukte die Karies zurück geht, die Bevölkerung in den Industriestaaten aber immer älter wird und die funktionsfähigen Zähne länger arbeiten müssen, wächst auch die Bedeutung von nicht kariogenen Zahnhartsubstanzverlusten.

Einige Darreichungsformen, die als geeignet beschrieben werden, die Mineralisation von Apatit auf der Zahnoberfläche anzuregen, sind bekannt. US 6,521,251 beschreibt eine Zusammensetzung, die neben Carbamidperoxid auch Calziumphosphate enthält, deren Löslichkeit etwas besser ist, als die von Apatit, wie Mono-Di-oder Trikalziumphosphat. Dennoch sind all diese Calziumphosphate nur wenig wasserlöslich, daher ist insbesondere bei den beschriebenen Zahnputzmitteln eher eine abrasive als eine remineralisierende Wirkung zu erwarten. Tatsächlich beschreibt US 5,851,514 unter anderem die Zugabe von Dikalziumphosphat als Abrasivum.

US 6,419,905 erwähnt die Zugabe von Kaliumsalzen (z. B. Citrat) und Fluorid zum Peroxid. Fluorid ist geeignet, um aus dem Speichel insbesondere Kalzium- und Phosphat-Ionen zu binden, wodurch Fluorapatit ausfällt. Wenn keine weiteren Ionen zugefügt werden, wird auch die Bildung von CaF₂ beobachtet. Die Calziumfluoridpartikel können in der Plaque gespeichert werden und über längere Zeit Fluorid abgeben, da sie löslicher sind, als der Apatit der Zahnhartsubstanz. Jedoch werden Zähne durch die gewissenhafte wiederholte tägliche Reinigung von Plaque weitgehend befreit. Damit ist die Wirkung von Calciumfluorid nur von kurzer Dauer und die fluoridhaltigen Produkte müssen regelmässig angewendet werden. Eine Bildung von neuem Apatit wurde mit solchen Produkten nicht beobachtet. Patent JP20000051804 beschreibt den parallelen Einsatz von konzentrierter Phosphorsäure, konz. H₂O₂ und Fluorapatitpulver. Problematisch erscheint dabei der Einsatz der konzentrierten Phosphorsäure, die gesunden Zahnschmelz merklich auflösen kann. Zudem ist die Bleichlösung stark reizend und darf nicht in Kontakt mit dem Zahnfleisch kommen, was aber in abgeschwächter Form für alle oxidativ wirkenden Zahnbleichmittel gilt. Zudem führt die wiederholte Anwendung nicht zum Aufbau einer Mineralisationsschicht.

Eine säurefreie Anwendung wird in US 20050281759 beschrieben. Als wesentlicher Inhaltsstoff wird hier Kalziumperoxophosphat vorgeschlagen. Kerngedanke ist hier, dass eine einzige Substanz aufhellend und remineralisierend wirken soll, da parallel zur Oxidation die Freisetzung von Kalzium- und Phosphat-Ionen ausgelöst wird. Nicht klar ist, ob die Salze in der verhältnismässig kurzen Einwirkungsdauer einen nennenswerten Apatitaufbau leisten können. US 6,303,104 beschreibt ein oxidationsmittelfreies Zwei-Komponentensystem aus löslichen Kalzium- und Phosphatsalzen, dass auch eine aufhellende Wirkung haben soll. Die Aufhellung soll durch die Zugabe von Natriumgluconat hervorgerufen werden, welches färbende Metallionen (z. B. Eisen) aus dem Zahnschmelz komplexiert. Beim Vermischen der Komponenten ist sofort mit einer Ausfällung der schwerlöslichen Kalziumphosphate zu rechnen und es ist nicht zu erkennen, warum es zu ausgeprägter Remineralisation kommen soll, zumal es sich bei dem Produkt um eine Zahncreme handelt, die nur wenige Minuten in Kontakt mit den Zahnflächen verbleibt. U.S. Pat. 6,102,050 beschreibt eine Zahnseide mit Titandioxidpartikeln, der eine aufhellende, remineralisierende und desensibilisierende Wirkung auf die Interdentalflächen zugeschrieben wird. Es sollen Titandioxidmikropartikel der Grösse 0.1 bis 1.5 µm sowohl als mildes Abrasivum wirken, als auch vom Schmelz absorbiert werden, was mit einer aufhellenden Wirkung verbunden sei. Vermutlich können sich die Partikel allenfalls mechanisch in geeignete Hohlräume einlagern, was keine stabile Verankerung verspricht und nur einen temporären Effekt haben kann.

Alle bisher beschriebenen Patente berücksichtigen nicht, dass Biominerale ihre hohe strukturelle Organisation und Stabilität nur erreichen, weil sie sich in Gegenwart von speziellen Biomolekülen bilden, die Mikro- und Makrostrukturierung vorgeben.

WO 2005/027863 beschreibt ein Zahnpflegemittel, dass über reinigende, remineralisierende, desensibilisierende und aufhellende Wirkung verfügen soll. Als aktive Komponente zur Remineralisation und Aufhellung wird ein nanoskaliges Apatit-Gelatine-Komposit vorgeschlagen, das in Gegenwart einer wässrigen Gelatinelösung gefällt wird und daher Polypeptide eingelagert hat. Dieses Material soll durch sogenannte "Neomineralisation" auf der Zahnoberfläche einen Schutzfilm von dentinähnlicher Struktur bilden können, der eine Oberflächenglättung bewirken soll und offene Dentintubuli verschliessen können soll. Diese Wirkung ist für eine Zahncreme nicht nachvollziehbar, da bevorzugt nur 0.01-2 Gew.-% "Nanite" (WO 01/01930) in diesem Zahnpflegemittel enthalten sind. Die Einwirkung der aktiven Substanzen liegt bei nur maximal einigen Minuten täglich. Eine ausgeprägte oder flächendeckende Mineralabscheidung wird bezweifelt. Eine Mineralabscheidung auf Schmelz ist zudem nicht beschrieben. Ein kontinuierlicher Zuwachs der Filmdicke bei längerer Anwendung des Pflegemittels wird ebenfalls nicht erwähnt. Zudem ist die poröse, wenig geordnete Struktur von Dentin nicht in der Lage, den Zahn vor korrosiven Angriffen zu schützen. Das kommerziell erhältliche Produkt Tooth Mousse oder Mi-Paste basiert auf Patentschriften von Reynolds [WO 98/40406] und soll porösen Schmelz remineralisieren. Die Erfindung beruht darauf, dass Casein (CPP) eine stabilisierende Wirkung auf amorphes Calciumphosphat (ACP) besitzt. Der Wirkstoff CPP-ACP soll in Kontakt mit Zahnhartsubstanz zu Hydroxylapatit remineralisieren. Ein solcher Schutzfilm von dentinartiger Struktur erscheint nicht geeignet, um einen dauerhaften Schutz zu bieten.

Allen Patenten ist gemein, dass sie nur von einer Remineralisation sprechen ohne diese zu belegen und/oder desensibilisierende Wirkung haben. US 2012/0027829A1 beschreibt die Bildung von Hydroxylapatitschichten (HAP) auf Dentin, wenn wiederholt pastenförmige Mischungen aus Propylenglykol, Glycerin, Xylitol, Polyethylenglykol, Cethylpyridiniumchlorid, Tetracalciumphosphat und ein Alkalisalz der Phosphorsäure auf die Zähne aufgebracht werden. Da Tetracalciumphosphat in Gegenwart von Wasser sofort mit den Salzen der Phosphorsäure reagiert, werden zuvor zwei getrennte Pasten hergestellt die erst kurz von der Anwendung gemischt werden. Eine Bildung von HAP auf Schmelz wird nicht beschrieben und es gibt keine Daten zur gebildeten Schicht, die bei eigenen Versuchen auch nicht nachstellbar war.

Die Möglichkeit einer, bei wiederholter Anwendung immer dicker werdenden Fluorapatit-Schicht mit Zahnschmelz-ähnlicher Festigkeit bietet die in US2005220724 und DE 10 2004 054 584.7 beschriebene Technik. Wasserlösliche Phospat- und Fluorid-Salze werden in dem gepufferten Gel A, Kalzium-Ionen im Gel B eingearbeitet. Optional durch eine ionenfreie Schutzschicht getrennt, werden die bei physiologischer Temperatur festen Gelatine-Gele unter Erwärmung nacheinander auf in-vitro Zahnoberflächen aufgetragen. In Abhängigkeit von den Austauschzyklen der Gele kann eine Zunahme der Schichtstärke beobachtet werden. Die Wachtumsgeschwindigkeiten betragen 0.5 bis 5.0 µm/Tag. Die biologischen Strukturen des Zahnmaterials werden individuell vom Fluorapatit abgebildet. Hohlräume durch offenliegende Dentintubuli werden nach einigen Austauschzyklen verschlossen.

Hinsichtlich einer Anwendung am Menschen erweist es sich als ungünstig, dass die Gele vor der Applikation erwärmt werden müssen. Durch das Aufbringen der zweiten und dritten Gelschicht können darunter liegende, bereits applizierte Gelschichten wieder verflüssigt werden und sich in unerwünschter Weise mit darüber liegenden Schichten vermischen. Insbesondere kleinere Darreichungsmengen trocknen bei Exposition an der Luft schnell aus und ein Verflüssigen durch Erwärmung ist dann nicht mehr ohne weiteres möglich. Die Methode erlaubt kaum, genau definierte Gelmengen gleicher Dicke auf den Zahn aufzubringen. Ferner tragen die drei bis zu 6 mm dicken Gelschichten stark auf, was bei Schutzsystemen wie Schienen oder Pflastern zu Problemen führt, da hier Platz für grosse Gelreservoirs geschaffen werden muss.

Ferner wird die Methode zunehmend aufwändig, wenn der gesamte Kiefer mit allen Zahnflächen behandelt werden soll. Da zur Bildung von Fluorapatit idealer Weise eine Anwendungsdauer von 8 Stunden nicht unterschritten werden sollte, wäre es von Vorteil, wenn der Patient das System selbst anwenden könnte, indem er es vor dem Schlafengehen anwendet. Dazu müsste er die Gele erwärmen und präzise auf den Zähnen platzieren, was sehr schwer möglich ist, da erwärmte flüssige Gelatine sehr klebrig ist. Zudem stören die Lippen bei Auftragen der flüssigen Gele. Zudem ist die Verbrennungsgefahr gross. Ein weiterer Nachteil ist, dass die Gele im Mundmilieu flüssig bleiben und nicht sicher auf den Zähnen haften.

Da die Gele trotz eines Schutzes wie z.B. einer individualisierten Tiefziehschiene auslaufen, muss die Schiene mit einem geeigneten Abdichtsystem abgedichtet werden, was die Methode noch komplizierter macht.

Die Verwendung von vorgefertigten Gelstreifen DE 10 2006 055 223 A1 hat den Vorteil, dass das aufwändige Erwärmen entfällt und die Streifen die gleiche Dicke haben. Ein grosser Nachteil ist allerdings, dass die Streifen nur Teilareale der Zähne erreichen. Da Erosionen aber mehr oder weniger alle Flächen der Zähne betreffen, wäre es wünschenswert, dass das Mineralisations-Kit auch überall wirken kann. Ferner ist es sehr umständlich die zwei Streifen auszupacken und beispielsweise in eine Tiefziehschiene einzubringen, die überdies noch ein Reservoir für die Gelstreifen haben muss. Ausserdem ist die Gefahr gross, dass die Gele in der falschen Reihenfolge auf den Zahn aufgebracht werden, wodurch das System unwirksam wird, oder die Streifen nicht plan- oder passgenau aufeinander gesetzt werden, wodurch sich die Wirkung vermindern kann. Auch ist das Problem der Abdichtung nicht zufriedenstellend gelöst. Da die Streifen sich unter den Bedingungen im Mundmilieu verflüssigen, ist eine Abdichtung aber notwendig, da die Wirkstoffe sonst in den Mundraum auslaufen können.

Aufgabe der Erfindung war die Bereitstellung Formulierungen und Systemen, die eine Applikation von Wirkstoffen, Verbindungen oder festen Ablagerungen, insbesondere kristallinen Feststoffen ermöglichen und, die dafür einen direkten und länger andauernden Kontakt zur Zahnoberfläche benötigen. Vorzugsweise sollte die Applikation oder Abscheidung vereinfacht und zugleich effizienter gestaltet werden. Darüber hinaus sollte die Möglichkeit eröffnet werden weitgehend alle Zahnflächen eines Kieferbogens, insbesondere Zahnbogens eines Unter- oder Oberkiefers zu erreichen, ohne dass die Produkte in die Mundhöhle auslaufen können. Mit dem System sollen vorzugsweise alternativ unterschiedlichste Zahnpflegeprodukte, Zahn-Kosmetika oder Zahn-Medizinprodukte, die überall an den Zähnen wirken sollen, applizierbar sein. Ferner soll die Abscheidung von Apatit auf den Zahnoberflächen angenehmer und effizienter erfolgen. Zudem soll die Abscheidung auf den Zahnoberflächen homogener ermöglicht werden und vorzugsweise ein Vertauschen von Mineralisationsmatrices unterbunden werden können.

Gelöst wird die Aufgabe mit einem dreidimensionalen Formkörper mit einer Matrix, dessen Matrix unter Mundbedingungen fest bleibt und mit Wirkstoffen angereichert ist. Die Wirkstoffe können retardiert über einen Zeitraum an die Zähne abgegeben werden. Der dreidimensionale Formkörper und damit die dreidimensionale Matrix hat dabei die Form des Negativs einer vereinfachten Zahnreihe oder eines Teils der Zahnreihe. Als Matrix können organische Gelbildner verwendet werden wie Agar-Agar, Carboxymethylcellulose, Polyacrylsäure, Silikonelastomere, Hydrogele, denaturiertes Kollagen (Gelatine) oder Mischungen aus diesen, welche gegebenenfalls durch chemische Fixation schwerlöslich unter Mundbedingungen gemacht werden.

Gelöst werden die Aufgaben daher durch eine Formulierung nach Anspruch 1, eine Applikationszahnschiene nach Anspruch 7 und ein Kit nach Anspruch 26 sowie durch die Verfahren zur Herstellung der Formulierung nach den Ansprüchen 19 und 20.

Gegenstand der Erfindung ist daher mindestens ein teilelastischer, bogenförmiger, dreidimensionaler Formkörper, umfassend eine Matrix aus Gel, wobei der Formkörper ausgewählt ist aus a) einem ersten dreidimensionalen Formkörper enthaltend eine Matrix umfassend mindestens ein Gel und b) einem weiteren dreidimensionalen Formkörper enthaltend eine zweite Matrix umfassend mindestens ein Gel, und die mindestens eine Matrix, insbesondere die eine und/oder die zweite Matrix, enthält jeweils unabhängig mindestens einen Wirkstoff, ein pharmakologisch verträgliches Salz, Solvat eines Wirkstoffs, oder ein mineralisierend wirkendes System.

Wobei jeder Formkörper jeweils unabhängig zumindest teilweise einem Zahnbogennegativ entspricht, und im Wesentlichen formschlüssig oder passgenau an mindestens einen Zahn oder mindestens teilweise an einem Zahnbogen aufbringbar ist, oder der Formkörper zu mindestens einem Zahn eines Zahnbogens beabstandet ist und jeder Formkörper unabhängig zumindest teilweise in mindestens einer Ebene, eine gegenüber wässrigen Medien verminderte Löslichkeit im Vergleich zur Matrix aufweist, wobei die Ebene, insbesondere Ebene, oder zumindest teilweisen Hülle als Membran fungieren.

Dabei ist es besonders bevorzugt, wenn der erste und/oder weitere Formkörper jeweils unabhängig zumindest teilweise mindestens eine an der äußeren Oberfläche angeordnete Ebene oder eine äußere Hülle aufweist. Die verminderte Löslichkeit kann durch eine chemische kovalente Vernetzung eines Gels und/oder durch eine Beschichtung der Formkörper eingestellt werden. Der Grad der Vernetzung und Beschichtung kann beliebig eingestellt werden.

Nach einer bevorzugten Ausführungsformen ist der Formkörper bogenförmig und weist eine Nut auf, die ausgebildet ist mindesten einen Zahn oder mindestens einen Teil eines Zahnbogen aufzunehmen oder der Formkörper liegt in Form einer Zahnschiene vor oder der Formkörper entspricht zumindest teilweise einem Zahnbogennegativ oder der Formkörper liegt formschlüssig oder passgenau an mindestens einem Zahn oder einem Teil eines Zahnbogens an oder entspricht zumindest teilweise einem Zahnbogennegativ oder ist beabstandet zu einem Zahnbogen. Die Zähne und Zahnbögen entsprechen denen von Wirbeltieren, insbesondere menschlichen. Bevorzugt sind bleibende Zähne oder auch Milchzähne.

Entsprechend einer besonders bevorzugten Ausführungsform der Erfindung umfasst der Formkörper folgende Gele: a) das Gel des weiteren Formkörpers umfasst wasserlösliche Phosphate oder zu wasserlöslichen Phosphat-Ionen hydrolysierbare Phosphate und weist einen pH-Wert von 2 bis 8 auf, und b) das Gel des ersten Formkörpers umfasst wasserlösliche Calcium-Ionen oder Calcium-Ionen freisetzende Verbindungen und weist einen pH-Wert von 3.5 bis 14 auf und a) das Gel des ersten Formkörpers und/oder b) das Gel des weiteren Formkörpers umfassen (i) einen Gehalt an Wasser oder eines Gemisches von Wasser und einem organischen Lösemittel, und (ii) optional mindestens eine Carbonsäure und/oder ein Puffersystem. Erfindungsgemäß enthält b) das Gel des weiteren Formkörpers mindestens ein wasserlösliches Fluorid oder eine Fluoride freisetzende Verbindung.

Die Matrix, insbesondere Mineralisationsmatrix oder auch Wirkstoffmatrix, Matrix zur Abgabe von Pflegeprodukten, Kosmetika basiert auf mindestens einem während der Herstellung formbaren und, insbesondere verfestigbaren Gel. Somit umfasst die Erfindung einen Formkörper umfassend mindestens ein Gel basierend auf einem organischen Gelbilder umfassend (a) denaturiertes Kollagen, Hydrokolloiden, Hydrogele, Polypeptide, Proteinhydrolysate, synthetische Polyaminosäuren, Polysacchariden, Polyacrylaten, Polyurethane, Casein, Stärkemehl, Cellulose, HPMC, Gummi Arabicum, Galactomannane, Guarkernmehl, Konjak, Xanthan, Calciumalginat, Dextran, Scleroglucan, Pektin, Carragenan (κ-, 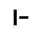 und λ-Carrageen), Agar-Agar, Alginat, Alginsäure, Natriumalginat, Calciumalginat, Traganth oder Mischungen umfassend mindestens zwei der genannten Gelbilder, oder anorganischen Gelbilder (b) umfassend Bentonite, Kieselgele oder Gemische enthaltend einen anorganischen Gelbilder, (c) Silikonelastomere, (d) pharmazeutische Matrixbildner, Cellulosen, insbesondere Carboxymethylcellulose, Acrylate, insbesondere Polyacrylsäuren als Homo, Co-Polymere, als Block-Co-Polymere oder statistische Co-Polymere, sowie auch Mischungen der Gelbilder (a), (b), (c) und/oder (d). Bevorzugt wird Gelatine der folgenden Qualitäten eingesetzt von Bloom 175 bis 300, oder höher, bevorzugt ist Gelatine Bloom 300 (Schweineschwarte). Insbesondere als zweite Mineralisationsmatrix und/oder zur Ausbildung der mindestens einen Ebene oder Hülle können vorzugsweise auch die Polysaccharide und/oder Polyacrylate (Eudragit) zur Anwendung kommen. In den Matrices wird bevorzugt Gelatine eingesetzt, die erfindungsgemäss mit einem Weichmacher wie Glycerin oder einem anderen Polyol versetzt ist. Die Zugabe des Weichmachers verbessert die Handlingseigenschaften der Gelatine.

Erfindungsgemäße Formkörper umfassen Matrices basierend auf Gelatine als Gel und vorzugsweise einem Weichmacher, bevorzugt ist ein Polyol, wie Glycerin und/oder deren Umsetzungsprodukte optional in Gegenwart von Wasser. Alternativ können auch Gelatine und ein Weichmacher wie Sorbitol eingesetzt werden. Der Weichmacher bewirkt eine Erhöhung des Schmelzbereiches durch Ausbildung von intermolekularen Wasserstoffbrückenbindungen. Als Gel wird erfindungsgemäss Gelatine eingesetzt, vorzugsweise (denaturiertes Kollagen, tierisches Eiweiss, Protein), besonders bevorzugt wird eine sauer hydrolysiertes Kollagen, oder Gelatine und ein Polyol, wie Glycerin.

Gleichfalls geeignet sind auch anorganische Gelbildner wie Bentonit oder Kieselgele. Die Formen können beispielsweise durch Gießen der erwärmten Matrix nach Anreichern mit den entsprechenden Wirkstoffen, in einem zweiteiligen Werkzeug hergestellt werden. Wirkstoffe können sein: mineralisierend wirkende Inhaltsstoffe wie Calcium-, Phosphat- und Fluorid-Salze, Bleichmittel, Antibiotika oder andere pharmakologisch wirksame Substanzen für den Zahnhalsbereich, plaquereduzierende Substanzen, karieshemmende Substanzen usw. Das Werkzeug erlaubt, Matrixformen herzustellen, die dem Negativ des vereinfachten Zahnbogens entsprechen. Dazu wird beispielsweise erwärmte Gelatine in die Negativ-Form des Kiefermodells eingebracht und das Werkzeug unter leichtem Druck mit dem zweiten Werkzeug auf der gegenüberliegenden Seite verschlossen. Die Abkühlung bewirkt eine Stabilisierung der ansonsten sehr weichen Matrix in der dreidimensionalen Form. Eine weitere Stabilisierung der Form wird durch chemische Vernetzung realisiert. Bezogen auf das konkret in einer parallelen Patentanmeldung beschriebene System zur Abscheidung von Apatit auf Zahnhartsubstanz lassen sich noch weitere Vereinfachungen in der Applikation erzielen:
Eine weitere Vereinfachung der Applikation wird dadurch erzielt, dass das dem Zahn abgewandte Calcium-haltige Gel bereits in eine Applikations-Zahnschiene synonym zu Schutzschiene eingebracht ist. Diese Anordnung lässt sich realisieren, indem zunächst die Zahnschiene in das Werkzeug eingebracht wird und anschließend die formbare Matrix umfassend das flüssige Gel eingetragen wird (Einlegeverfahren). Großtechnisch lässt sich das z.B. mittels 2-K-Injection-Molding-Verfahren realisieren. Bei diesem Verfahren können Zahnschiene bzw. Schutz-Schiene und Formkörper, d.h. Gelschiene direkt hintereinander in einer Form hergestellt werden. Ebenfalls möglich ist das Einbringen der formbaren Matrix als erwärmes Gel in bereits fertig produzierte Applikations-Zahnschienen durch Extrudieren. Ferner können gegossene Gelplatten durch leichtes Erwärmen in die nötige Form gebracht werden. Besonders geeignet sind thermoverformbare bzw. thermisch verformbare Zahn-, Schutz-Schienen wie in WO 2009045857 A1 und WO 2009029886 A1 beschrieben. Auf die Offenbarung der WO 2009045857 A1 und WO 2009029886 A1 wird vollständig Bezug genommen, insbesondere auf die dort genannten thermisch verformbaren Polymere und die Geometrie der Zahnschienen, die zum Inhalt dieser Patentanmeldung gemacht werden. Für den Patienten vereinfacht sich die Anwendung dadurch stark. Es sind nur noch die Schritte 1-3 nötig:
   1. Mundspülen mit der Vorbehandlung
   2. Einsetzen der Phosphat-Gel-Schiene
   3. Einsetzen der mit Calcium-Gel imprägnierten Schutzschiene synonym zu Applikations-Zahnschiene mit Matrix als eine Einheit.

Mit der Erfindung ist es möglich, lösliche Wirkstoffe auf den Zahn aufzubringen, ohne dass diese sofort abgespült werden. Als Schutz reicht eine individualisierbare Schiene ohne weitere Abdichtung. Insbesondere kann ein komplexes System zur Abscheidung von Apatit auf natürlicher Zahnhartsubstanz deutlich komfortabler anwendbar zu machen. Die Mineralisation kann nahezu überall an den Zähnen wirken und erreicht nicht nur Teilareale der Zähne. Die einfache Anwendung eröffnet die Möglichkeit, dass sowohl Zahnärzte an Patienten als auch Patienten selbst das System anwenden können. Das Applikationssytem kann grundsätzlich in der Einlegetechnik oder im 2K-Spritzguß realisiert werden.

Die Abscheidung von Apatit erfolgt bevorzugt , indem die auf Basis von denaturiertem Kollagen oder anderen Gelbildnern und Mineralstoffen zusammengesetzte Matrix, insbesondere jeweils Mineralisationsmatrix, durch chemische Modifikation im Mundmilieu unlöslich wird, insbesondere als eine Formulierung mit getrennten Formkörpern oder als mehrteiliger Formkörper umfassend jeweils mindestens eine oder zwei Mineralisaitionsmatrices Überraschend wird die Mineralisationsaktivität trotz der Modifizierung positiv beeinflusst, da das Gel im Mundmilieu nicht mehr verläuft und während der gesamten Behandlungsdauer wirken kann. Diese chemische Modifikation erfolgt vorzugsweise durch zumindest teilweise oberflächliche chemische Vernetzung der Mineralisationsmatrix, so dass mindestens eine teilweise chemisch vernetzte Ebene oder teilweise Hülle gebildet wird. Vorzugsweise ist die mindestens eine Ebene eine Oberfläche der Mineralisationsmatrix, bevorzugt bilden die Ebenen eine äussere Hülle an den Oberflächen der Mineralisationsmatrix aus. Erfindungsgemäss wirken die vernetzten Ebenen oder die Hülle wie eine Membran, durch die wässrige Medien, wie Speichel, in die Mineralisationsmatrix eindringen können und zugleich eine Abscheidung von Apatit ausserhalb der Mineralisationsmatrix auf den Zahnoberflächen erfolgen kann, die mit der mindestens einen Ebene oder der Hülle der Mineralisationsmatrix in Kontakt ist. Die gebildete Ebene oder Hülle kann sich erfindungsgemäss einerseits durch ihre sehr dünne Schicht optimal bei einer Quellung im Mund an die Oberflächen der Zähne anlegen und begünstigt auch die Abscheidung von Apatit im Bereich der Zahnzwischenräume. Die Mineralisationsmatrix wird zudem bei Körpertemperatur flexibler und kann sich optimal an die Zahnkonturen anlegen. Ein weiterer besonderer Vorteil der oberflächlichen Vernetzung der Mineralisationsmatrix, bei der sich ein Formkörper ausbildet, ist, dass ein zumindest teilelastischer Formkörper gebildet wird, der sich optimal der Oberflächenkontur von Zähnen und Zahnreihen anschmiegen kann. Besonders gut liegt der teilelastische Formkörper durch die Quellung der Mineralisationsmatrix im Mundmilieu an den Zahnoberflächen an. Um den Formkörper, umfassend mindestens eine Mineralisationsmatrix, optimal an den bukkalen, labialen, mesialen und/oder approximalen Oberflächen der Zähne zu fixieren, wird vorteilhaft der mindestens eine Formkörper in eine Applikations-Zahnschiene eingelegt bzw. in der Zahnschiene bereitgestellt. Durch eine Fixierung mit einer Zahnschiene gelingt es mit dem erfindungsgemässen Formkörper auch, auf mindestens einem Teil oder nahezu vollständig auf den Zähnen sowohl des Oberals auch des Unterkiefers bukkal, mesial, labial, palatinal, distal sowie approximal Apatit abzuscheiden. Durch den erfindungsgemässen mindestens einen Formkörper kann auf einfache Weise erstmalig einfach durch Einsetzen der Formkörper in eine Applikations-Zahnschiene und Aufsetzen der Schiene auf die Zähne über Nacht bspw. für etwa 8 bis 12 Stunden, optional bis 16 oder 24 Stunden, alternativ auch am Tag eine biomimetische Remineralisierung von mindestens teilweise grösser gleich 1 µm erfolgen, bevorzugt grösser gleich 2 µm, bevorzugt grösser gleich 3 µm, besonders bevorzugt ist eine biomimetische Reminerasilierung von mindestens teilweise, vorzugweise im Mittel, von 1 bis 10 µm, 1 bis 5 µm. Besonders bevorzugt erfolgt die Remineralisierung in der Fläche mit einer möglichst homogen Schichtdicke.

Überraschender Weise wurde festgestellt, dass sich das Mineralisationsprodukt nach der chemischen Stabilisierung gleichmässiger und dichter auf der Zahnoberfläche bildet. Die vernetzte Ebene oder Hülle ist so porös, dass trotz Ausbildung der Hülle vergleichbare Apatitschichten abgeschieden werden können, wie erfindungsgemässn Beispiele aufzeigen. Es wird davon ausgegangen, dass sich durch die chemische Modifizierung ein weniger temperaturempfindliches und vorzugsweise weniger hydrolyseempfindliches Netz aus Polypeptiden um die Mineralisationsmatrix bildet, dessen Poren jedoch noch gross genug sind, um Moleküle und Ionen durchzulassen, welche die stabile, geordnete Apatitschicht an der Zahnoberfläche bilden. Die chemisch vernetzte Schicht oder Ebene wirkt quasi wie eine Ionen und moleküldurchlässige Membran.

Eine ausreichend dicke, homogene und stabile Apatitschicht lässt sich durch das optimale Zusammenspiel der Komponenten: Mineralsalze, Puffer und pH-Wert erzielen. Ausschlaggebend sind die korrekt gewählten Konzentrationen bei entsprechendem Vernetzungsgrad. Die erfindungsgemässe Formulierung kann zur Abscheidung von Apatit auf Zähnen oder jeglichen anderen biologischen Oberflächen wie auf einer Knochenmatrix eingesetzt werden.

Die einfachere Anwendung der erfindungsgemässen Formulierungen eröffnet die Möglichkeit, dass sowohl Zahnärzte an Patienten als auch Patienten selbst das System anwenden können. Die Abscheidung einer fluoridreichen Calciumphosphatschicht kann Sensibilitäten an den Zähnen vermindern, sie kann Risse und initiale Porösitäten reduzieren und erhöht die Säurestabilität der Zähne. Initiale Zahnhartsubstanzverluste infolge von Säureerosion können gestoppt bzw. teilweise oder vollständig rückgängig gemacht werden. Der erhöhte Fluoridgehalt im Vergleich zu unbehandelten Zähnen in den Schichten reduziert die Löslichkeit des neu gebildeten Minerals. Der natürliche Schmelz wird durch die Schutzschicht vor Zahnbürstenabrasion geschützt.

Gegenstand der Erfindung sind Formkörper, die geeignet sind zur biomimetischen Abscheidung von Apatit ausgewählt aus Fluorapatit (Ca₅[F|(PO₄)₃), Hydroxylapatit (Ca₅[F|(PO₄)₃) oder deren Gemischen auf Zähnen oder auch auf einer Knochenmatrix von Wirbeltieren, wobei der Formkörper mindestens einen teilelastischen, dreidimensionalen und bogenförmigen Formkörper aufweist, umfassend mindestens eine Matrix, insbesondere eine Mineralisationsmatrix, enthaltend mindestens ein Gel, und wobei der Formkörper vorzugsweise zumindest teilweise in mindestens einer Ebene eine gegenüber wässrigen Medien verminderte Löslichkeit im Vergleich zur Matrix, insbesondere Mineralisationsmatrix, aufweist, wobei die Ebene als Membran fungiert, und
a) die mindestens eine Mineralisationsmatrix ein Gel enthaltend wasserlösliche Phosphate oder zu wasserlöslichen Phosphat-Ionen hydrolysierbare Phosphate und einen pH-Wert von 2 bis 8 aufweist (Phosphat-Matrix), und
b) die mindestens eine oder eine zweite Mineralisationsmatrix ein zweites Gel mit einem pH-Wert von 3.5 bis 14 aufweist, umfassend Calcium-Ionen oder Calcium-Ionen freisetzende Verbindungen (Calcium-Matrix),
besonders bevorzugt liegt die mindestens eine Mineralisationsmatrix in einem ersten Formkörper I. und die zweite Mineralisationsmatrix in einem weiteren Formkörper II. vor, alternativ liegen zwei Mineralisationsmatrices in einem Formkörper vor.

Entsprechend einer bevorzugten Ausführungsform der Erfindung liegen die mindestens eine oder die zweite Matrices, insbesondere Mineralisationsmatrices jeweils unabhängig umfassend mindestens ein Gel in Form eines bogenförmigen Formkörpers, insbesondere ersten und/oder weiteren Formkörpers, bevorzugt als mindestens teilweisen Kiefernegativs vor. Bevorzugt ist es, wenn die genannte mindestens eine Ebene eine an der äusseren Oberfläche angeordnete Ebene oder eine im Wesentlichen vollständige äussere Hülle ist.

Erfindungsgemässe Formulierungen umfassen einen ersten oder weiteren Formkörper in Form eines bogenförmigen, teilelastischen Formkörpers mit jeweils einer einschliessenden Hülle mit verminderter Löslichkeit, die insbesondere als Membran fungiert. Die vorgenannten Formkörper liegen vorzugsweise mit Schichtdicken der seitlichen Wände, die jeweils unabhängig voneinander definierte Schichtdicken aufweisen vor, vorzugsweise in Form von U-, J-, L-förmigen Profilen. Die seitlichen Wände liegen in der Regel labial, bukkal und/oder distal an den Zähnen an. Die Schichtdicken liegen vorteilhaft im Bereich von 5 bis 10.000 µm, vorzugsweise von 10 bis 6000 µm, besonders bevorzugt sind Mineralisationsmatrices mit Schichtdicken von 5 bis 3000 µm, bevorzugt 100 bis 600 µm, insbesondere 300 bis 600 µm oder um 500 µm plus/minus 100 µm, insbesondere. plus/minus 50 µm. Die erfindungsgemässen Formkörper umfassen zumindest teilweise eine Hülle oder mindestens eine Ebene mit verminderter Löslichkeit. Der Formkörper liegt vorzugsweise in Form zumindest eines teilweisen Zahnbogennegativs oder Kiefernegativs vor, vorzugsweise als eine Negativform des mindestens einen Zahns oder von Zähnen des Ober- und/oder Unterkiefers. Formkörper der Calcium-Matrix liegen vorzugswiese mit einer Schichtdicke von 500 bis 3000 µm, insbesondere 500 bis 1500 µm vor, besonders bevorzugt von 500 bis 1200 µm oder um 1000 µm plus/minus 200 µm, insbesondere plus/minus 100 µm. Formkörper der Phosphat-Matrix liegen vorzugsweise mit einer Schichtdicke von 100 bis 3000 µm, insbesondere von 100 bis 1000 µm vor, besonders bevorzugt sind 150 bis 800 µm, bevorzugt 300 bis 800 µm, insbesondere 400 bis 600 µm oder um 500 µm plus/minus 200 µm, insbesondere plus/minus 100 µm, insbesondere plus/minus 50 µm.

Erfindungsgemässe Formkörper weisen nach der Vernetzung und wahlweise nach anschliessender Trocknung einen Wassergehalt von 8 bis 60 Gew.-%, bevorzugt von 30 bis 55 Gew.-% auf. Weiter bevorzugt weisen die Gele der Erfindungsgemässe Formkörper nach der Vernetzung und wahlweise nach anschliessender Trocknung in der Mineralisationsmatrix der Phosphat-Matrix einen Wassergehalt von 20 bis 40 Gew.-%, bevorzugt von 25 bis 35 Gew.-%, besonders bevorzugt um 30 Gew.-% mit einer Abweichung von plus/minus 5 Gew.-% auf. Entsprechend bevorzugt sind Formulierungen der Calcium-Matrix, die nach der Vernetzung und wahlweise nach anschliessender Trocknung in der Mineralisationsmatrix einen Wassergehalt von 30 bis 60 Gew.-%, bevorzugt von 40 bis 60 Gew.-%, vorzugsweise um 50 Gew.-% mit einer Abweichung von plus/minus 5 Gew.-% aufweisen. Die so hergestellten Formkörper können anschliessend luft- und feuchtigkeitsdicht in Blister, Sachets oder dergleichen eingeschweisst werden.

Die Hülle oder die Ebene verhindert oder vermindert ein Auflösen des jeweiligen Formkörpers umfassend die Matrix bzw. Mineralisationsmatrix im Mundmilieu. Jedoch kann H₂O aus dem Speichel eindringen, und Fluoride, Calcium- und Phosphat-Ionen sowie Komposite oder Polypeptide können durch die Hülle diffundieren und auf der Zahnoberfläche als Apatit, Hydroxylapatit oder Fluorapatit abgeschieden werden. Die Hülle umschliesst eine wasserreiche Gelatinematrix aus der die Komposite, ionenbeladene Wassermoleküle bzw. hydratisierte Ionen und/oder Wirkstoffe heraus diffundieren können. Die Ebenen oder die Hülle fungiert als Membran für chemische Verbindungen durch die Wirkstoffe, pharmakologisch wirksame Substanzen, Pflegeprodukte, Komposite bspw. aus Polypeptiden und Salzen, sowie für Salze und Wasser etc. herausdiffundieren können. Die Diffusion der Komposite ist wichtig, denn die Komposite sind mit organischen Makromolekülen substituierte Hydroxyapatite, die den Zahnschmelz bilden und, um diese auf den Zahnoberflächen abzuscheiden. Erfindungsgemäss werden Fluorapatit-Protein-Komposite aus den Formkörpern auf den Zahnoberflächen abgeschieden. Unter einer gegenüber wässrigen Medien verminderten Löslichkeit der chemisch vernetzten Ebene oder Hülle im Vergleich zur Mineralisationsmatrix wird folgendes verstanden: Die chemische unvernetzte Mineralisationsmatrix und optional nur mit einem Weichmacher modifizierte Mineralisationsmatrix, bspw. die mit Glycerin über Wasserstoffbrückenbindungen vorzugsweise als Addukt vernetzte Gelatine.

Erfindungsgemäss bilden die mindestens eine Ebene oder die Hülle die äussere Begrenzung mindestens einer Matrix wie Mineralisationsmatrix. Die Ebene oder Hülle kann auf unterschiedliche Weise erhalten werden, indem eine chemische Vernetzung der Mineralisationsmatrix durchgeführt wird oder durch Auftragen einer Beschichtung (coating) auf die Mineralisationsmatrix zur Ausbildung einer Ionen und Wasser durchlässigen Oberfläche der Mineralisationsmatrix, die als Membran fungiert. Die Vernetzung oder die Beschichtung können durch Tauchen, Auftragen, wie Pinseln, Sprühen, Rollen sowie durch weitere, dem Fachmann bekannte Massnahmen erfolgen. Die Ausbildung der Ebenen oder der Hülle kann auch mit unregelmässigen oder regelmässigen Durchbrechungen bzw. Poren oder unter Zugabe von Porenbildnern erfolgen.

Der aus der erfindungsgemässen Formkörpern abgeschiedene Fluorapatit liegt vorzugsweise auf den Zähnen kristallin vor, bevorzugt microkristallin, besonders bevorzugt in Form von nadeligen Kristalliten. Die innerhalb eines Anwendungszyklus von etwa 8 Stunden auf den Zahnoberflächen abgeschiedene zumindest teilweise Apatitschicht, vorzugsweise durchgängige Apatitschicht, weist mindestens eine Schichtdicke von 2 µm auf. Ebenso im Rahmen der Erfindung sind zumindest teilweise nicht durchgängige Apatitschichten, die unregelmässig bis vorzugsweise homogen zumindest einen Teil der behandelten Zahnoberfläche bedecken. Die Apatitschichten können zumindest teilweise bis zu 15 µm betragen, vorzugsweise werden im Wesentlichen vorzugsweise homogene Apatitschichten von größer gleich 2 µm, vorzugsweise größer gleich 5 µm bis 13 µm, und im Mittel von 2 bis 10 µm erhalten.

Unter Zähnen von Wirbeltieren werden erfasst menschliche Zähne, Prothesen menschlicher Zähne, Milchzähne (*Dentes decidui*), Bleibende-Zähne (*Dentes permanentes*), Kronen, Inlays, Implantate, Zähne von Tieren, wie Haustieren und Nutztieren wie Hunden, Pferden, Katzen.

Unter einem mindestens teilelastischen Formkörper wird im Sinne der Erfindung ein dreidimensionaler Formkörper verstanden, der elastische Eigenschaften aufweist.. Erfindungsgemäß ist der Formkörper bogenförmig, insbesondere in Form eines mindestens teilweisen Kiefernegativs, und weist elastische Eigenschaften auf. Als elastisch oder teilelastisch gilt der Formkörper, wenn der Körper unter Krafteinwirkung seine Form verändert und bei Wegfall der einwirkenden Kraft zumindest teilweise oder vollständig wieder in die Ursprungsform zurückkehrt. Die elastische oder teilelastische Eigenschaft weist der Formkörper vorzugsweise bei der Anwendung im Mundmilieu auf und vorzugsweise nach der Herstellung. Bei zu langer Trocknung kann die Elastizität abnehmen.

In einem nach der Erfindung besonders bevorzugten weiteren Formkörper weist die Mineralisationsmatrix in a) folgende Zusammensetzung auf: a) die mindestens eine Mineralisationsmatrix weist ein Gel auf, umfassend (i) wasserlösliche Phosphate oder zu wasserlöslichen Phosphat-Ionen hydrolysierbare Phosphate, insbesondere Na₂HPO₄, vorzugsweise liegt der Gehalt an Phosphat in der Mineralisationsmatrix bei 1 bis 10 Gew.-%, vorzugsweise von 2 bis 8 Gew.-%, besonders bevorzugt von 5 bis 8 Gew.-% (ii) einen Gehalt an Wasser oder ein Gemisch von Wasser und einem organischen Lösemittel, (iii) optional mindestens eine Carbonsäure, insbesondere eine Hydroxycarbonsäure, wie Milchsäure, und/oder ein Puffersystem, insbesondere liegt ein Puffersystem zur Einstellung des pH-Wertes im Bereich von 2 bis 8 vor, insbesondere von 3.5 bis 8, bevorzugt von 3.5 bis 6, besonders bevorzugt um 5,5 plus/minus 0,5. Der Gehalt bezieht sich auf PO₄³⁻.

Gleichzeitig weist der nach der Erfindung besonders bevorzugte ersten Formkörper eine Mineralisationsmatrix in b) folgender Zusammensetzung auf: die zweite Mineralisationsmatrix oder die mindestens eine Mineralisationsmatrix weist ein zweites Gel auf, umfassend (i) Calcium-Ionen oder Calcium-Ionen freisetzende Verbindungen, insbesondere Calciumdichlorid oder Hydrate davon, vorzugsweise zusätzlich Calciumsulfat, Nanoapatit, Natriumcarbonat oder Calciumoxalat, vorzugsweise liegt der Gehalt an Calcium in der Mineralisationsmatrix bei 1 bis 10 Gew.-%, vorzugsweise größer gleich 1,5 bis 7,5 Gew.-%, (ii) optional Wasser oder ein Gemisch von Wasser und einem organischen Lösemittel, und (iii) optional mindestens eine Carbonsäure, wie eine Hydroxycarbonsäure bspw. Milchsäure, und/oder ein Puffersystem. Zur Herstellung der Puffer werden bevorzugt Fruchtsäuren und Alkali-Salze eingesetzt. Der Gehalt bezieht sich auf das Calcium (Ca²⁺).

Weiter ist es bevorzugt, wenn die Formulierung in der mindestens einen Matrix ein Gel aufweist, umfassend mindestens ein wasserlösliches Fluorid (F⁻), mit Fluorid-Ionen, oder eine Fluoride freisetzende Verbindung. Besonders bevorzugt weist der weitere Formkörper in a) als weitere Komponente (iv) mindestens ein wasserlösliches Fluorid oder eine Fluoride freisetzende Verbindung auf. Entsprechend einer Alternative kann die Matrix des ersten Formkörper Fluoride als Wirkstoff zur Fluoridierung der Zähne umfassen, hier dient der Formkörper als System zur Abgabe eines Wirkstoffs und insbesondere nicht zur Abscheidung von Apatit.

Nach einer bevorzugten Ausgestaltung der Erfindung umfasst das mindestens eine wasserlösliche Fluorid oder die mindestens eine Fluoride freisetzende Verbindung, insbesondere in a) die mindestens eine Mineralisationsmatrix, (i) mindestens eine unsubstituierte oder substituierte Alkyl-Gruppe aufweisende quaternäre mono- oder poly-Ammonium-Verbindung, vorzugsweise mit vier substituierten Alkyl-Gruppen, wobei die mindestens eine substituierte Alkyl-Gruppe umfasst Hydroxyalkyl-, Carboxyalkyl-, Aminoalkyl-Gruppen mit 1 bis 25 C-Atomen oder organofunktionelle durch Heteroatome unterbrochene Gruppen mit bis zu 50 C-Atomen. Bevorzugte Ammonium-Verbindungen können 1 bis 20 quaternäre Ammonium-Funktionalitäten enthalten, vorzugsweise 1, 2, 3, 4, 5, 6, 7, 8 Ammonium-Funktionalitäten, bevorzugt wird Olaflur (N,N,N'-Tris(2-hydroxyethyl)-N'-octadecyl-1,3-diaminopropandihydro-fluorid) als wasserlösliches Fluorid eingesetzt. Gleichfalls bevorzugt sind Aminfluoride, wie Oleaflur, Decaflur, Ethanolamin-Hydrofluorid, (ii) eine Fluoride freisetzende organofunktionelle Amino-Verbindung oder ein Fluoride freisetzendes Antiseptikum auf Basis von organofunktionellen Amino-Verbindungen, wie insbesondere Fluoride von *N*-Octyl-1-[10-(4-octyliminopyridin-1-yl)decyl]pyridin-4-imin, Cetylpyridinium fluorid, oder c) wasserlösliche anorganische Fluoride, wie Alkalifluoride, Natriumfluorid, Kaliumfluorid, Zinnfluorid, Ammoniumfluorid, oder Fluoride freisetzende anorganische Fluoride, wie Zinkfluorid, Zinkhydroxyfluorid.

Die chemische Vernetzung zur Ausbildung der chemisch mindestens teilvernetzten Ebene oder teilvernetzten Hülle, insbesondere kovalent vernetzt, die eine gegenüber wässrigen Medien verminderte Löslichkeit als die entsprechende nicht auf diese Art chemisch vernetzte Matrix aufweist, erfolgt vorzugsweise durch Vernetzung mit Vernetzern wie Dialdehyden, bevorzugt sind alle äusseren Oberflächen der Mineralisationsmatrix chemisch mindestens teilvernetzt. Die lediglich intermolekulare Ausbildung von Wasserstoffbrücken durch die Anwesenheit von Glycerin in der Gelatine verbesserte die Handlingseigenschaften und thermische Stabilität der Gelatine. Erfindungsgemässe Formkörper können auch eine Oberflächentextur, die den Zahnoberflächen eines Zahnbogens nachempfunden ist, aufweisen.

Als Verbindungen zur chemischen Vernetzung, insbesondere kovalenten Vernetzung, in der mindestens einen Ebene oder zur Ausbildung der Hülle der Matrix werden die di- oder polyfunktionellen Vernetzer eingesetzt, bevorzugt Glutardialdehyd. Die chemischen Vernetzer bilden mit den Gelen, insbesondere den Polypeptiden oder Polyaminosäuren kovalente Vernetzungstellen aus. Vorzugsweise umfassen die di- oder polyfunktionellen Vernetzer Dialdehyde, Polyepoxide, und/oder Polyisocyanate sowie Mischungen umfassend mindestens zwei Vernetzer. Weiter bevorzugt werden pharmakologisch unbedenkliche Vernetzer eingesetzt. Bevorzugte Dialdehyde umfassen alpha, omega-Dialdehyde von Kohlenwasserstoffen, insbesondere umfassend 2 bis 50 C-Atome, insbesondere 4 bis 10 C-Atome in der bifunktionellen Alkylen Gruppe. Durch eine Behandlung der Mineralisationsmatrix mit einem Vernetzer verringert sich die Löslichkeit der Gelatine bis auf ein Niveau, dass sie für etwa 8 Stunden im Mundmilieu nicht verflüssigt. Bevorzugt ist die Behandlung mit Glutardialdehyd für mindestens 5 s, je nach Anwendung kann die Vernetzung länger erfolgen und damit stärker sein, bspw., wenn eine Mineralisationsmatrix für 12 bis 16 Stunden im Mundmilieu verbleiben soll. Nach 40 s Spülen in einer 0.5 Gew.-%igen Glutardialdehyllösung reduziert sich die Löslichkeit des Gels so weit, dass es bei Mundmillieu für bis zu 8 Stunden nicht verflüssigt. Die optional verwendbaren Membran(schichten) sind ionenfrei.

Die Vernetzungslösung weist vorzugsweise einen Gehalt von etwa 0,25 bis 0,5 Gew.-% Vernetzer, vorzugsweise Glutardialdehyd auf. Die besten Ergebnisse hinsichtlich einer ausreichenden Vernetzung der Formkörper und optimalen Durchlässigkeit für die abzuscheidenden Apatitkomposite hat sich eine Behandlungszeit von, 0 bis 60 s, bevorzugt von etwa 5 bis 40 s, besonders bevorzugt von 10 bis 30 s, erfindungsgemäß um 20 Sekunden (s) gezeigt. Entsprechend einer bevorzugten Ausführungsform der Erfindung ist ein Gegenstand der Erfindung ein Applikationssystem zur Zahnbehandlung von Wirbeltieren, insbesondere zur Zahnbehandlung vom einem oder mehreren menschlichen Zähnen, umfassend mindestens eine Applikations-Zahnschiene, mit mindestens einem Formkörper entsprechend der erfindungsgemäßen Offenbarung, wobei die Applikations-Zahnschiene geformt und/oder geeignet ist, mindestens einen an die Schiene angepassten ersten dreidimensionalen Formkörper enthaltend eine Matrix aufzunehmen. Erfindungsgemäße ist die Applikations-Zahnschiene thermisch verformbar. Entsprechend einer bevorzugten Ausführungsform des Applikationssystems ist in den ersten Formkörper mindestens ein weiterer dreidimensionaler Formkörper einsetzbar ist, wobei der weitere Formkörper eine zweite Matrix enthält. Das Applikationssystem umfasst vorteilhaft einen ersten Formkörper und einen weiteren Formkörper, wobei der weitere Formkörper ausgebildet ist zumindest einen Zahn oder einen Teil eines Zahnbogens aufzunehmen, wenn die Applikations-Zahnschiene von einem Wirbeltier angewendet wird.

Die erfindungsgemäße Applikations-Zahnschiene liegt vorzugsweise in Form eines U-förmigen, J-förmigen oder L-förmigen Profils vor, insbesondere ist die Schiene entlang des Profils bogenförmig, oder die Applikations-Zahnschiene ist ausgewählt aus einer bogenförmigen Zahnschiene mit Nut, die vorzugsweise ausgebildet ist mindesten einen Zahn oder mindestens einen Teil eines Zahnbogens aufzunehmen, die entspricht Zahnschiene zumindest teilweise einem Zahnbogennegativ, die Zahnschiene ist zumindest teilweise beabstandet zu dem teilweisen Zahnbogen, die Zahnschiene ist zumindest teilweise beabstandet zu dem teilweisen Zahnbogen und weist ein Reservoir zur Aufnahme des ersten und/oder weiteren Formkörpers auf, die Zahnschiene entspricht mindestens einem Zahn oder die Zahnschiene entspricht zumindest teilweise einem Kiefernegativ.

Nach einer bevorzugten Ausführungsform der Erfindung bilden in dem Applikationssystem die Applikations-Zahnschiene und der erste Formkörper eine Einheit, vorzugsweise sind der erste Formkörper und die Zahnschiene fest miteinander verbunden. Die Matrix des ersten Formkörpers umfasst in dieser Ausführungsform vorteilhaft Wirkstoffe oder bildet die Calcium-Matrix. Die Calcium-Matrix kann später mit dem weiteren Formkörper umfassend die Phopshat-Matrix zur eine Applikationssystem zur biomimetischen Abscheidung von Apatit, insbesondere Fluorapatit zusammengesetzt werden.

Erfindungsgemäß umfasst die Matrix der Applikations-Zahnschiene, die Matrix des ersten Formkörpers oder die Matrix des weiteren Formkörpers mindestens einen Wirkstoff oder ein mineralisierend wirkendes System mit Phosphaten, Calcium und Fluoriden, pharmakologisch wirksame Substanzen umfassend Antibiotika, Antiseptika, die Mundschleimhaut aufbauenden Wirkstoff, Bleichmittel, plaquereduzierende Substanz, karieshemmende und/oder kariesvermindernde Substanz umfasst.

Sowohl der Formkörper alleine als auch der in dem Applikationssytem einsetzbare Formkörper ist ausgewählt aus einem ersten und weiteren Formkörper, wobei jeweils unabhängig der Formkörper bogenförmig ist und eine Nut aufweist, die ausgebildet ist mindesten einen Zahn oder mindestens einen Teil eines Zahnbogens aufzunehmen, der Formkörper in Form einer Zahnschiene vorliegt, der Formkörper zumindest teilweise einem Zahnbogennegativ entspricht, der Formkörper formschlüssig oder passgenau anliegt oder zumindest teilweise beabstandet zu dem teilweisen Zahnbogen oder mindestens einem Zahn ist, der Formkörper zumindest teilweise einem Kiefernegativ entspricht.

Entsprechend einer erfindungsgemäßen Alternative umfasst das Applikationssytem alternativ (i) a) eine Applikations-Zahnschiene, die einen ersten dreidimensionale Formkörper enthaltend eine Matrix aufnimmt und b) der erste dreidimensionale Formkörper einen weiteren Formkörper aufnimmt enthaltend eine Matrix, optional getrennt durch eine Membranschicht, oder nach einer Alternative bilden (ii) a) die Applikations-Zahnschiene und der erste Formkörper eine Einheit, wobei b) der erste dreidimensionale Formkörper einen weiteren Formkörper aufnimmt enthaltend eine Matrix, optional getrennt durch eine Membranschicht, oder (iii) a) die Applikations-Zahnschiene umfasst eine Matrix und nimmt einen ersten dreidimensionalen Formkörper enthaltend eine Matrix auf; optional getrennt durch eine Membranschicht.

Die Applikations-Zahnschiene des Applikationssystem ist zumindest teilweise aus einem Polymer geformt, umfassend thermoplastische Polymere, Elastomere, Thermoplast, thermoplastische Elastomere, duroplastische Polymere und/oder aus einer Mischung enthaltend mindestens zwei der vorgenannten Polymere sowie optional weitere übliche Füllstoffe, Rheologiemodifizierer, Additive, Vernetzer und/oder Katalysatoren. Weiter bevorzugt sind (i) thermoplastischen Polymere umfassen Homo- und/oder Copolymere umfassend Acrylnitril-Butadien-Styrol (ABS), Acrylnitril-Ethylen-Butadien-Styrol, Polyamide (PA), Polylactat (PLA), Polymethylmethacrylat (PMMA), Polycarbonat (PC), Polyethylenterephthalat (PET), Polyethylen (PE), Polyethylen hoher Dichte (HDPE), Polyethylen niedriger Dichte (LDPE), Polyethylen ultra-niedriger Dichte (ULDPE),Polypropylen (PP), Polystyrol (PS), Polyetheretherketon (PEEK), Ethylenvinylacetat (EVA), (ii) die Elastomere bildende Polymere umfassen Silikone, Alignat, Kautschuke, Naturkautschuk und Silikonkautschuk, oder (iii) thermoplastische Elastomere umfassen thermoplastische Elastomere auf Olefinbasis, PP/EPDM, Vernetzte thermoplastische Elastomere auf Olefinbasis, wie PP/EPDM, Urethane, thermoplastische Polyesterelastomere, Copolyester, , Styrol-Blockcopolymere wie SBS, SEBS, SEPS, SEEPS und MBS, thermoplastische Copolyamide sowie Mischungen umfassend mindesten zwei der vorgenannten Verbindungen.

Beispiele für Elastomere umfassen natürliche Gummis, Polyisoprene, der ButylKautschuke, Isobutylen und Isopren-Copolymere, halogenierten Butylkautschuke, Chlorbutyl-Kautschuk, Brombutyl-Kautschuken, Polybutadienen, Polystyrol und Polybutadien-Copolymere, Nitril-Kautschuken, hydrierten Nitril-Kautschuke Polybutadien und Acrylnitril-Copolymere, Chloropren-Kautschuken, Polychloropren, Neopren, Polyethylen gesättigten Kautschuken, Ethylen-Propylen-Kautschuken und Polypropylen-Copolymeren, Ethylen-Propylen-Dien-Kautschuken, Epichlorhydrin-Kautschuken, Polyacryl-, Silikon-, Fluorsilikon-Kautschuk, Perfluorelastomeren, Polyether-Block-Amide, Tetrafluorethylen / Propylen-Kautschuken, chlorsulfoniertes polyethelenes, Ethylen- Vinylacetate, thermoplastische Elastomere, thermoplastische Vulkanisate, Polyurethan-Kautschuken, Polysulfid-Kautschuken, Silikonen, Polyorganosiloxane, Kombinationen davon und dergleichen. In einer Ausführungsform kann das Elastomer ein Silikon sein. Silikon-Materialien sind im Allgemeinen komprimierbar und damit weicher. Bei Körpertemperatur sind Silikone formbeständig, zäh, flexibel und belastbar. Andererseits haben Silikone den Vorteil dünne, aber formstabile Formkörper oder optional auch Applikationsschienen oder Teile davon, wie Innenfütterungen zu bilden. Zudem sind Silikone relativ stabil und nehmen wenig Feuchtigkeit auf. Silikone umfassen Homo-und Copolymere, die mindestens ein Siloxan aus Dimethylsiloxanen, Methylphenylsiloxane, Methylvinylsiloxanen, Methylfluoroalkylsiloxanes, Methylethylidennorbornen-siloxanes enthalten. Bevorzugt Polyorganosiloxane umfassen Silikone, Polydimethylsiloxane, Polymethylphenylsiloxane, Polydialkylsiloxane, Polymethylvinylsiloxane, Polymethylfluoroalkylsiloxane, Polymethylethylidennorborn-nesiloxane oder Mischungen enthaltend diese. Zusätzlich kann die Membran aus üblichen Materailien in dentalen Anwendungen hergestellt sein, wie Polyolefine, EthylenVinylacetat-Copolymer (EVA), Ethylen-Vinylalkohol-Copolymer (EVAL), Polycaprolacton (PCL), Polyvinylchlorid (PVC), Polyester, Polycarbonat, Polyamid, Polyurethan, Polyesteramid, Celluloseethern, Ethyl Cellulose, Cellulose Propyl, Isopropyl Cellulose, Cellulose Butyl, t-Butyl, Celluloseacetat, Polyvinylacetat, Polyvinylalkohol, Schellack, chemische oder lichthärtende Materialien (z. B. Methacrylat oder Acrylat-Harze), Kombinationen davon und dergleichen. Geeignete Polyolefine zur Herstellung der Applikations-Zahnschieneumfassen Polyethylen (PE), Polyethylen hoher Dichte (HDPE), Polyethylen niederer Dichte (LDPE), Polyethylen ultraniedriger Dichte (ULDPE), Polypropylen (PP), und Polytetrafluorethylen (PTFE) (zB TEFLON). Vinylterminierten Siloxanpolymer Mischungen sind typischerweise Wärme gehärtet. Sie enthalten Benzoylperoxid, die als eine radikalische Polymerisation und Vernetzungsinitiator wirken.

In einer Ausführungsform kann der Formkörper Hydroxyl-terminiertes Polydimethylsiloxan umfassen, die durch Kondensationsreaktion ausgehärtet. Als Katalysator oder Vernetzungsmittel können Tetraethylsilikat, und einen Katalysator, wie z. B. Dibutylzinndilaurat zugesetzt werden. Das vernetzte Polysiloxan ist ein hochelastisches Elastomer.

Ein erfindungsgemäßes Applikationssystem umfasst (i) den weiteren Formkörper enthaltend eine Matrix, wobei der weitere dreidimensionale Formkörper zumindest teilelastisch und insbesondere bogenförmig ist, und die (a) Matrix (Phosphat-Matrix) umfasst, (a1) mindestens eine Mineralisationsmatrix umfassend mindestens ein Gel, umfassend, (a2) mindestens ein wasserlösliches Phosphat oder zu wasserlöslichen Phosphat-Ionen hydrolysierbare Phosphate, und (a3) optional mindestens eine Carbonsäure und/oder ein Puffersystem, (a4) optional wasserlösliche Fluoride oder eine Fluoride freisetzende Verbindung, (a5) optional einen Gehalt an Wasser oder ein Gemisch von Wasser und einem organischen Lösemittel, und/oder (ii) den ersten Formkörper enthaltend eine Matrix, und der erste dreidimensionale Formkörper zumindest teilelastisch und insbesondere bogenförmig ist, und die (b) Matrix (Calcium-Marix) umfasst (b1) mindestens eine Mineralisationsmatrix umfassend mindestens ein Gel, umfassend, (b2) wasserlösliche Calcium-Ionen oder Calcium-Ionen freisetzende Verbindungen, und (b3) optional mindestens eine Carbonsäure und/oder ein Puffersystem, (b4) optional Wasser oder ein Gemisch von Wasser und einem organischen Lösemittel, oder der erste Formkörper umfasst Matrices und ist zumindest teilelastisch, wobei die (c) Matrices umfassen (c) teilelastische Formkörper C umfasst (c1) mindestens eine Mineralisationsmatrix umfassend mindestens ein Gel, enthaltend, (c1.1) mindestens ein wasserlösliches Phosphat oder zu wasserlöslichen Phosphat-Ionen hydrolysierbares Phosphat, und (c1.2) optional mindestens eine Carbonsäure und/oder ein Puffersystem, (c1.3) optional wasserlösliches Fluoride oder eine Fluoride freisetzende Verbindung, (c1.4) optional einen Gehalt an Wasser oder ein Gemisch von Wasser und einem organischen Lösemittel, (c2) optional eine Membran(schicht), und (c3) mindestens eine Mineralisationsmatrix umfassend mindestens ein Gel, enthaltend, (c3.1) wasserlösliche Calcium-Ionen oder Calcium-Ionen freisetzende Verbindungen, und (c3.2) optional mindestens eine Carbonsäure und/oder ein Puffersystem, (c3.3) optional Wasser oder ein Gemisch von Wasser und einem organischen Lösemittel, wobei der Formkörper den Schichtaufbau c1 und c3 oder c1, c2 und c3 aufweist, wobei c3 an der Applikations-Zahnschiene anliegt und c1 innen angeordnet ist, insbesondere um bei einer dentalen Anwendung an einem Wirbeltier mit mindestens einem Zahn oder einem Teil eines Zahnbogens in Kontakt zu treten.

Ebenso umfasst die Erfindung ein Applikationssystem, wobei in einer Alternative (i) die Applikations-Zahnschiene einen ersten dreidimensionale Formkörper enthaltend eine Matrix aufweist und der erste Formkörper eine Einheit mit der Applikations-Zahnschiene bildet, wobei (b) die Matrix umfasst, (b1) mindestens eine Mineralisationsmatrix umfassend mindestens ein Gel, (b2) wasserlösliche Calcium-Ionen oder Calcium-Ionen freisetzende Verbindungen, und (b3) optional mindestens eine Carbonsäure und/oder ein Puffersystem, (b4) optional Wasser oder ein Gemisch von Wasser und einem organischen Lösemittel, ist oder (ii) der erste Formkörper (b) umfassend die Mineralisationsmatrix (b1) mit (b2), (b3) und optional (b4) in die Applikations-Zahnschiene einsetzbar ist.

Ferner ist ein Gegenstand der Erfindung ein Applikationssystem, wobei in einer Alternative (i) die Applikations-Zahnschiene einen ersten Formkörper umfasst in den ein weiterer Formkörper enthaltend eine Matrix einsetzbar ist, wobei der weitere Formkörper dreidimensional ist, und die (a) Matrix umfasst (a1) mindestens eine Mineralisationsmatrix umfassend mindestens ein Gel, enthält, umfassend, (a2) mindestens ein wasserlösliches Phosphat oder zu wasserlöslichen Phosphat-Ionen hydrolysierbare Phosphate, und (a3) optional mindestens eine Carbonsäure und/oder ein Puffersystem, (a4) optional wasserlösliches Fluoride oder eine Fluoride freisetzende Verbindung, (a5) optional einen Gehalt an Wasser oder ein Gemisch von Wasser und einem organischen Lösemittel, oder (ii) der weitere Formkörper (a) umfassend die Mineralisationsmatrix (a1) mit (a2), (a3), optional (4a) und optional (a5) optional getrennt durch eine Membran als Einheit mit der Applikations-Zahnschiene wie vorstehend offenbart (i) vorliegt.

Die Carbonsäuren werden vorzugsweise ausgewählt aus Fruchtsäuren, wie α-Hydroxycarbonsäuren wie Äpfelsäure, Zitronensäure, Glycolsäure, Milchsäure und Weinsäure; Aminosäuren, Fettsäuren, Hydroxycarbonsäuren, Dicarbonsäuren und Mischungen umfassend mindestens zwei der genannten Säuren und/oder das Puffersystem umfasst Carboxylate von Alkylcarbonsäuren, Fettsäuren, Fruchtsäuren, Fumarate, Aminosäuren, Hydroxycarbonsäuren, Dicarbonsäuren und Mischungen umfassend mindestens zwei der genannten Säuren oder Phosphatpuffer. Für die Puffersysteme werden vorteilhaft Alkali- und/oder Erdalkali-Salze oder Zink-Salze eingesetzt.

Die Puffersysteme umfassen EDTA, TRIS: Tris(hydroxymethyl)-aminomethan für pH 7,2 bis 9,0, HEPES: 4-(2-Hydroxyethyl)-1-piperazinethanesulfonsäure für pH 6,8 bis 8,2, HEPPS: 4-(2-Hydroxyethyl)-piperazin-1-propansulfonsäure für pH 7,3 bis 8,7, Barbital-Acetat Puffer, MES: 2-(N-Morpholino)ethansulfonsäure für pH 5,2 bis 6,7, Kohlensäure-Bicarbonat-System für pH 6,2 bis 8,6; neutral, Kohlensäure-Silicat-Puffer für pH 5,0 bis 6,2; schwach sauer, Essigsäure-Acetat-Puffer für pH 3,7 bis 5,7, Phosphatpuffer: NaH₂PO₄ + Na₂HPO₄ für pH 5,4 bis 8,0, Ammoniakpuffer NH₃ + H₂O + NH₄Cl für pH 8,2 bis 10,2, Zitronensäure- oder Zitratpuffer. Besonders bevorzugte Puffersysteme umfassend Milchsäure Puffersysteme, EDTA, oder Barbital-Acetat Puffer und in der Mundspüllösung TRIS (Tris(hydroxymethyl)-aminomethan) Puffer. In der Mundspüllösung synonym zu Vorbehandlungslösung wird TRIS (Tris(hydroxymethyl)-aminomethan) eingesetzt.

Erfindungsgemäss einsetzbare Phosphate zur Herstellung der Phosphat enthaltenden Mineralisationsmatrices umfassen die Phosphate, Hydrogenphosphate, oder zu wasserlöslichen Phosphat-Ionen hydrolysierbare Phosphate umfassend a) Alkaliphosphate, Erdalkaliphosphate, Dihydrogenphosphate, Natriumdihydrogenphosphat, NaH₂PO₄, Kaliumdihydrogenphosphat, KH₂PO₄, Hydrogenphosphate, Dikaliumhydrogenphosphat, K₂HPO, Dinatriumhydrogenphosphat, Na₂HPO₄, Phosphatester, Monoester, Diester und Triester von Phosphaten, Natriumphosphat, Na₃PO₄, Kaliumphosphat, K₃PO₄, Calciumdihydrogenphosphat, Ca(H₂PO₄)₂, Monoester, Diester und Triester Calciumhydrogenphosphat, CaHPO₄, Calciumphosphat, Ca₃(PO₄)₂ und/oder b) die Calcium-Ionen oder Calcium-Ionen freisetzende Verbindungen umfasst Calciumchlorid, Calciumdichlorid-Dihydrat, Calcium-Salz einer Carbonsäure umfassend Alkylcarbonsäuren, Hydroxycarbonsäure, Dicarbonsäuren, Fruchtsäuren, Aminosäuren, wie Calciumlactat, Calciumgluconat, Calciumlacto-Gluconat, Calciumalginat, Calcium-L-Ascorbat, in Wasser schlecht lösliche Calcium-Ionen retardiert freisetzende Verbindungen umfassend Calciumsulfat, Calciumapatit, Calciumcarbonat, Calciumoxalat, Calciumphosphat, Calciumalginat, vorzugsweise mit einer Partikelgrösse von kleiner 100 µm, bevorzugt um 10 µm, besonders bevorzugt kleiner gleich 5 µm bspw. bis 1 µm oder 50 nm oder vorzugsweise Gemische von wasserlöslichen und schlecht in Wasser löslichen Calcium-Ionen oder Calcium-Ionen freisetzenden Verbindungen. Die in Wasser schlecht löslichen Calcium-Ionen retardiert freisetzenden Verbindungen werden zur Texturverbesserung der teilweise klebrigen Gele dem gut wasserlösliche Calcium-Ionen enthaltendem Gel zugesetzt. In Bezug auf die Gesamtzusammensetzung der Mineralisationsmatrix umfassend das Gel können 1 bis 50 Gew.-% Wasser schlecht lösliche Calcium-freisetzende Verbindungen eingesetzt werden, bevorzugt werden 5 bis 30 Gew.-% eingesetzt.

Entsprechend einer Ausführungsform der Erfindung wird eine Formulierung zur Herstellung einer wässrigen Vorbehandlungslösung synonym zu Mundspüllösung zur Vorbehandlung der Zähne offenbart enthaltend mindestens ein gut wasserlösliches Calcium-Salz, umfassend vorzugsweise Calciumlactat, Calciumchlorid, Calciumgluconat, Calciumlacto-Gluconat, ein Hydrat der Salze oder eine Mischung enthaltend mindestens zwei der Salze, optional einen Gehalt eines Puffersystems, insbesondere TRIS (Tris(hydroxymethyl)-aminomethan), optional einen Gehalt an Maskierungsmittel oder Aromatisierungsmittel sowie üblichen Formulierungshilfsmitteln zur Herstellung einer Vorbehandlungslösung oder zusammen mit Wasser als Vorbehandlungslösung vor einer Behandlung in der Apatit auf Zähnen von Wirbeltieren abgeschieden wird. In Mundspüllösung (Vorbehandlungslösung) wird TRIS (Tris(hydroxymethyl)-aminomethan) eingesetzt. Gegenstand der Erfindung ist auch eine Formulierung in Form einer wässrigen Mundspüllösung umfassend Wasser, 0,01 bis 2 mol eines gut wasserlöslichen Calcium-Salzes in Bezug auf die Gesamtzusammensetzung, optional 0,01 bis 0,5 mol, vorzugsweise zu 0,05 bis 0,2 mol eines Puffersystems, insbesondere TRIS (Tris(hydroxymethyl)-aminomethan), optional Maskierungsmittel oder Aromatisierungsmittel und aufweisend einen pH-Wert von 5.0 bis 12.0. Die Mundspüllösung bzw. Vorbehandlungslösung kann auch zur Behandlung einzelner Zähne eingesetzt werden.

Nach einer weiteren Alternative wird eine Formulierung einer wässrigen Mundspüllösung offenbart, vorzugsweise zur Verwendung mit einer vorgenannten erfindungsgemässen Formulierung, umfassend Wasser, 0.1 bis 30 Gew.-% eines gut wasserlöslichen Calcium-Salzes, insbesondere 5 bis 20 Gew.-%, bevorzugt 5 bis 15 Gew.-% in Bezug auf die Gesamtzusammensetzung, vorzugsweise umfassend Calciumlactat, Calciumchlorid, Calciumgluconat, Calciumlacto-Gluconat, ein Hydrat der Salze oder eine Mischung enthaltend mindestens zwei der Salze, optional mit einem Gehalt eines Puffersystems, insbesondere Tris, optional Maskierungsmittel oder Aromatisierungsmittel und aufweisend einen pH-Wert von 5.0 bis 12.0.

Gleichfalls Gegenstand der Erfindung ist ein Verfahren zur Herstellung des Formkörpers sowie ein Formkörper erhältlich nach dem Verfahren, indem eine formbare Matrix in ein erstes Werkstück, insbesondere mit einer Ausnehmung, die zumindest einem teilweisen Kiefernegativ oder teilweisen Zahnbogennegativ entspricht oder davon abgeleitet ist, eingebracht wird und mit einem weiteren Werkstück, das zumindest teilweise einem Kieferpositiv oder teilweisen Zahnbogenpositiv entspricht, unter Normaldruck oder Überdruck geformt wird, optional abgekühlt wird, nach Formung wird die geformte Matrix als Formkörper entnommen, wobei der erste oder der weitere Formkörper erhalten wird. Die Herstellung der formbaren Matrices ist nachfolgend beschrieben. Anschließend kann die Vernetzung durchgeführt werden.

Gleichfalls Gegenstand der Erfindung ist ein Verfahren zur Herstellung des Applikationssystem sowie ein Applikationssystem erhältlich nach dem Verfahren, indem (a) eine Applikations-Zahnschiene (a.1) vorgelegt oder (a.2) geformt wird, insbesondere mittels Spritzguss, Extrusion oder andere dem Fachmann bekannte Verfahren, die Zahnschiene wird vorzugsweise aus den vorgenannten Polymeren, wie Polyolefine, geformt, (b.1) der erster Formkörper in die Applikations-Zahnschiene (b.1.1) vorgeformt eingebracht wird, insbesondere mittels Spritzguss der formbaren Matrix geformt wird, und in die Applikations-Zahnschiene eingebracht wird, oder (b.1.2) insbesondere die formbare Matrix, mittels 2K-Spritzguss in die Applikations-Zahnschiene eingebracht und geformt wird oder (b1.3) der erste Formkörper wird vom Anwender geformt und in die Applikations-Zahnschiene eingebracht.

Ebenso wird eine Verfahren offenbart, indem (c.1) der weiterer Formkörper in die Applikations-Zahnschiene (c.1.1) vorgeformt eingebracht wird, vorzugsweise wird die formbare Matrix geformt, vorzugsweise mittels Spritzguss, und in den ersten Formkörper in der Applikations-Zahnschiene eingebracht oder (c.1.2) insbesondere wird die formbare Matrix mittels 2K-Spritzguss optional getrennt durch eine Membran, in den ersten Formkörper eingebracht, oder (c.1.3) der weitere Formkörper wird vom Anwender geformt und in die den ersten Formkörper in der Applikations-Zahnschiene eingebracht.

Erfindungsgemäße wird in dem Verfahren der erste und/oder der weitere Formkörper hergestellt, indem die formbare Matrix in ein erstes Werkstück zumindest eines teilweisen Kiefernegativs oder teilweisen Zahnbogennegativs eingebracht wird und mit einem weiteren Werkstück, das zumindest teilweise einem Kieferpositiv oder teilweisen Zahnbogenpositivs entspricht, unter Normaldruck oder Überdruck geformt wird, optional abgekühlt wird, nach Formung wird die geformte Matrix als Formkörper entnommen, wobei das erste Werkstück optional die Applikations-Zahnschien enthält, wenn der erste Formkörper hergestellt wird und, wobei optional das weitere Werkstück ebenfalls eine Applikations-Zahnschiene bei der Herstellung des ersten Formkörpers enthält.

Ferner ist Gegenstand der Erfindung ein Verfahren zur Herstellung der Formkörper oder der Formkörper zur Verwendung mit der Applikationszahnschiene, indem
a) der weitere Formkörper umfassend eine Matrix umfassend mindestens ein Gel enthaltend mindestens ein wasserlösliches Phosphat oder Phosphat freisetzende Verbindung, Gelatine und Glycerin, optional mindestens eine Carbonsäure und/oder ein Puffersystem, optional wasserlösliche Fluoride oder eine Fluoride freisetzende Verbindung, optional einen Gehalt an Wasser oder ein Gemisch von Wasser und ein organisches Lösemittel hergestellt wird, indem,
   - als formbare Matrix, insbesondere warmes, nicht verfestigtes Gel in das erste Werkzeug eingebracht wird, unter Druck gepresst wird, optional wird abgekühlt, das verfestigte Gel wird als Matrix oder als Formkörper umfassend die Matrix entnommen, vorzugsweise wird nachfolgend die Matrix an mindestens einer äußeren Ebene chemisch vernetzt oder außen zu einer Hülle chemisch vernetzt oder beschichtet und/oder
b) der erste Formkörper umfassend eine Matrix umfassend mindestens ein Gel enthaltend wasserlösliche Calcium-Ionen oder Calcium-Ionen freisetzende Verbindungen, optional mindestens eine Carbonsäure und/oder ein Puffersystem, optional Wasser oder ein Gemisch von Wasser und einem organischen Lösemittel hergestellt wird, indem
   - als formbare Matrix, insbesondere warmes nicht verfestigtes Gel in das zweite Werkzeug eingebracht wird, unter Druck gepresst wird, optional wird abgekühlt, das verfestigte Gel wird als Matrix oder als Formkörper entnommen, vorzugweise wird nachfolgend die Matrix an mindestens einer äußeren Ebene chemisch vernetzt oder außen zu einer Hülle chemisch vernetzt oder in einer Ebene oder mit einer Hülle beschichtet.

Die Herstellung des Formkörpers der Phosphat-Matrix erfolgt, indem a) zur Herstellung mindestens der Matrix enthaltend das Gel, auch Phosphatkomponente A genannt, wird in einem ersten Schritt eine Mischung von (i) 0.05 bis 4 mol/l, 0.5 bis 1.5 mol/l wasserlöslichen Phosphaten oder zu wasserlöslichen Phosphat-Ionen hydrolysierbaren Phosphaten, (ii) eine entsprechende Menge Wasser oder ein Gemisch von Wasser und einem organischen Lösemittel, (iii) optional mindestens eine Carbonsäure und/oder ein Puffersystem, insbesondere zur Einstellung des pH-Wertes von 2 bis 8, vorzugsweise 3.5 bis 8, bevorzugt 3.5 bis 6, besonders bevorzugt um 5,5 plus/minus 0,5, (iv) 0 bis 6000 Gew.-ppm wasserlösliches Fluorid oder eine Fluoride freisetzende Verbindung, insbesondere 1 bis 4000 Gew.-ppm, weiter bevorzugt 500 bis 2500 Gew.-ppm, besonders bevorzugt um 2000 Gew.-ppm plus/minus 500 Gew.-ppm, gemischt, in einem weiteren Schritt wird aus der in a) hergestellten Mischung, b) mit Gelatine und optional Glycerin unter Erwärmen das Gel hergestellt, unter Erhalt des formbaren Matrix, anschließend erfolgt c) die Formung der Matrix, insbesondere Mineralisationsmatrix, und optional Verfestigung, und in einem optional nachfolgenden Schritt d) erfolgt eine Ausbildung einer an der äusseren Oberfläche angeordneten Ebene, insbesondere der Hülle, der mindestens einen Mineralisationsmatrix unter Bildung des Formkörpers.

Die Ebene oder Hülle kann durch Vernetzung oder Auftragen eines Coatings (Beschichtung) erzeugt werden. Zur Vernetzung der mindestens einen an der äusseren Oberfläche angeordneten Ebene der mindestens einen Mineralisationsmatrix, insbesondere in Form eines Flächenelements oder mindestens teilweisen Kiefernegativs, wird diese in einem weiteren Schritt mit einer Mischung, vorzugsweise einer wässrigen Lösung, enthaltend einen di- oder polyfunktionellen Vernetzer in Kontakt gebracht.

Die Phosphat-Lösung zur Herstellung der Phosphatkomponente A enthält unter anderem ein wasserlösliches Phosphat-Salz. Geeignet sind z.B. Alkalisalze wie Natrium-oder Kaliumphosphate, Hydrogen- oder Dihydrogenposphate. Die Aufzählung ist einschliesslich aber nicht ausschliesslich. Die Konzentration der Phosphatsalze in der Lösung beträgt zwischen 0.05 bis 4 mol/l Gel, bevorzugt 0.5 bis 1.5 mol/l, besonders bevorzugt um 1 mol/l plus/minus 0,5 mol/l. Die Phosphatlösung enthält ausserdem ein wasserlösliches Fluoridsalz z.B. ein Alkalisalz, oder Zinnfluorid oder Olafluor. Die Aufzählung ist einschliesslich aber nicht ausschliesslich. Die Konzentration des Fluorids liegt in der Lösung zwischen 0 bis 6000 Gew.-ppm, bevorzugt 200 bis 4000 Gew.-ppm, besonders bevorzugt 2500 bis 4000 Gew.-ppm oder um 3000 Gew.-ppm plus/minus 500 Gew.-ppm. Die Phosphat-Lösung kann durch Zugabe des Vernetzers, beispielsweise Glutardialdehyd, als Vernetzerlösung verwendet werden.

Der pH-Wert der Phosphat-Lösung liegt zwischen 2.0 und 8.0, bevorzugt zwischen 3.5 und 5.5 wird mit einem geeigneten Puffersystem eingestellt. Besonders geeignet sind Carbonsäuren wie Ascorbinsäure. Brenztraubensäure, Weinsäure, Essigsäure, Milchsäure oder Äpfelsäure aber auch alle anderen Puffersysteme. Die Konzentration des Puffers liegt zwischen 0.25 und 4.0 mol/l, bevorzugt zwischen 0.5 und 1.5 mol/l.

Aus der Lösung wird ein Gelatine-Glycerin-Gel hergestellt. Die Menge an Gelatine beträgt vorzugsweise 25 bis 40 Gew.-% und die Menge an Glycerin 5 bis 20 Gew.-% in Bezug auf die Gesamtzusammensetzung an wässrigem Gel. Um die Komponenten homogen zu mischen, wird die Zubereitung auf 40 bis 90°C erwärmt bevorzugt auf 50 bis 70°C. Die Dicke der Phosphat-Komponente A beträgt hierbei 50 bis 3000 µm, bevorzugt 200 bis 2000 µm, besonders bevorzugt 300 bis 1500 µm.

Die Herstellung des Formkörpers der Calcium-Matrix erfolgt, indem
a) zur Herstellung mindestens des Formkörpers umfassend eine Matrix enthaltend das Gel, auch Calciumkomponente B genannt, in einem ersten Schritt eine Mischung von (i) 0.1 bis 2 mol/l Calcium-Ionen oder Calcium-Ionen freisetzende Verbindungen, (ii) eine entsprechende Menge Wasser oder ein Gemisch von Wasser und einem organischen Lösemittel, (iii) optional mindestens eine Carbonsäure und/oder ein Puffersystem, insbesondere zur Einstellung des pH-Wertes von 3.5 bis 14, bevorzugt von 4.0 bis 6.0 oder 6.0 bis 11.0, vorzugsweise 4.0, besonders bevorzugt um 4.0 plus/minus 0,5, gemischt werden, in einem weiteren Schritt wird aus der in a) hergestellten Mischung b) mit Gelatine und optional Glycerin unter Erwärmen das Gel hergestellt, unter Erhalt der formbaren Matrix, anschließend erfolgt die c) Formung der formbaren Matrix, optional Verfestigung, unter Erhalt des Formkörpers und in einem optional nachfolgenden Schritt d) erfolgt die Ausbildung einer an der äusseren Oberfläche angeordneten Ebene, insbesondere der Hülle, der mindestens einen Mineralisationsmatrix unter Bildung des Formkörpers. Die Ebene oder Hülle kann durch die vorgenannte Vernetzung oder Auftragen eines Coatings erzeugt werden. Entscheidend bei der Herstellung der Ebene oder der Hülle ist eine gewisse Porosität, um eine Abscheidung der Biokomposite zu ermöglichen.

Zur Herstellung der vorgenannten Formulierungen werden in b) jeweils unabhängig im weiteren Schritt 5 bis 50 Gew.-% Gelatine in Bezug auf die Gesamtzusammensetzung Gel zugesetzt und 0 bis 30 Gew.-% Glycerin in Bezug auf die Gesamtzusammensetzung Gel zugesetzt, bevorzugt werden 25 bis 40 Gew.-% Gelatine und 5 bis 20 Gew.-% Glycerin zur Herstellung der Formulierung enthaltend wasserlösliche Phosphate oder zu wasserlöslichen Phosphat-Ionen hydrolysierbaren Phosphate enthaltenden Matrix, und 20 bis 40 Gew.-% Gelatine und 15 bis 25 Gew.-% Glycerin zur Herstellung der Formulierung enthaltend Calcium-Ionen oder Calcium-Ionen freisetzende Verbindungen enthaltenden Mineralisationsmatrix zugesetzt.

Die jeweiligen Gelatinen in Schritt b) werden vorzugsweise auf 40 bis 90 °C erwärmt, um die Komponenten homogen zu vermischen, bevorzugt ist der Temperaturbereich von 50 bis 70 °C um die formbaren Matrices herzustellen.

Die Lösung zur Herstellung der Calciumkomponente oder des Formkörpers enthält ein wasserlösliches Calciumsalz, z.B. Calciumchlorid oder Calciumlactat oder Calciumgluconat oder Calciumlacto-Gluconat. Die Aufzählung ist einschliesslich aber nicht ausschliesslich. Die Konzentration liegt zwischen 0.1 und 2.0 mol/l, bevorzugt zwischen 0.5 und 1.5 mol/l. Der pH-Wert zwischen 4.0 und 14.0, bevorzugt zwischen 6.0 und 11.0 wird mit einem geeigneten Puffersystem eingestellt. Besonders geeignet sind Carbonsäuren wie Ascorbinsäure, Brenztraubensäure, Weinsäure, Essigsäure, Milchsäure oder Äpfelsäure aber auch alle anderen Puffersysteme mit geeignetem pks-Wert. Die Konzentration des Puffers liegt zwischen 0.1 und 3.0 mol/l, bevorzugt zwischen 0.25 und 1.0 mol/l. Aus der Lösung wird ein Gelatine-Glycerin-Gel hergestellt. Die Menge an Gelatine beträgt vorzugsweise 20 bis 40 Gew-% in Bezug auf die Gesamtzusammensetzung an wässrigem Gel und die Menge an Glycerin 15 bis 25 Gew-%. Da die Calcium-Gelatine-Lösung auch nach Gelieren sehr klebrig ist und damit unangenehm im Handling wird zur Texturverbesserung ein schlecht lösliches Calciumsalz zugegeben. Besonders geeignet sind Calciumsulfat, Calciumapatit, Calciumcarbonat, Calciumoxalat. Die Aufzählung ist einschliesslich aber nicht ausschliesslich. Um eine besonders homogene Paste zu erhalten, ist es vorteilhaft wenn die Partikelgrössen unter 10 µm liegen. Bevorzugt werden Partikel mit Partikelgrössen kleiner 1 µm eingesetzt. Die Zugabe des schwer löslichen Calciumsalzes liegt bevorzugt bei 1 bis 50 %, sehr bevorzugt bei 5 bis 30%. Um die Komponenten homogen zu mischen, wird die Zubereitung unter Rühren auf 40-90°C erwärmt bevorzugt auf 50 bis 70°C.

Die Dicke des ersten Formkörpers oder der Calcium-Matrix beträgt hierbei 10 bis 3000 µm, bevorzugt 100 bis 1500 µm, besonders bevorzugt 300 bis 1500 µm, vorzugsweise 500 bis 1500 µm.
Zur Herstellung der Formkörper werden die noch nicht verfestigten Gele als formbare Matrices geformt und anschliessend verfestigt. Daher ist ebenfalls Gegenstand der Erfindung ein Verfahren, indem das im weiteren Schritt b) hergestellte Gel als formbare Matrix geformt wird, das verfestigbar ist. Nach der Vernetzung und wahlweise anschliessender Trocknung auf einen Wassergehalt zwischen 8 bis 60 Gew.-%, bevorzugt 30 bis 55 Gew.-% können die Formkörper entnommen werden. Die Trocknung stabilisiert die Formkörper zusätzlich. Der Grad der Vernetzung kann über die Art und Konzentration des Vernetzungsmittels, dem pH-Wert der Vernetzungslösung und die Einwirkzeit eingestellt werden.

Ebenfalls Gegenstand der Erfindung ist ein Applikationssystem umfassend eine Applikations-Zahnschiene, die vor oder während der Anwendung durch ein Wirbeltier thermisch verformbar ist
Gegenstand der Erfindung ist auch ein Kit umfassend ein Applikationssytem umfassend eine Applikations-Zahnschiene, einen ersten Formkörper und/oder einen weiteren Formkörper sowie optional eine Vorspüllösung.

Des Weiteren ist ein Gegenstand der Erfindung die Verwendung des erfindungsgemäßen Applikationssystems oder Kits zur biomimetischen Abscheidung von Apatit, von Fluorapatit, Behandlung von Lumen in Zähnen, Behandlung von Karies, von Entzündungen, zur Plaquereduktion, zum Bleichen der Zähne, zur Verabreichung von pharmakologisch wirksamen Substanzen, zur indiviuellen Medikation, zur Remineralisierung von durch Oxidation gebleichter Zähne. Gleichfalls bevorzugt ist die Verwendung eines Applikationssystem oder Kits zur Abscheidung von Apatit auf Oberflächen, insbesondere von Fluorapatit, mit einer Schichtdicke von zumindest teilweise größer gleich 1 µm, vorzugsweise größer gleich 2 µm, innerhalb von 16 Stunden, insbesondere bei Körpertemperatur im Mundmilieu oder bei 37 °C und einer Luftfeuchtigkeit von 95 %.

Ferner ist Gegenstand der Erfindung die Verwendung eines Applikationssystems oder Kits enthaltend Phosphat oder zu wasserlöslichen Phosphat-Ionen hydrolysierbare Phosphate, wasserlösliches Fluoride oder eine Fluoride freisetzende Verbindung und wasserlösliche Calcium-Ionen oder Calcium-Ionen freisetzende Verbindungen für eine remineralisierende Behandlung von Zähnen durch ein- bis mehrmalige, insbesondere ein- bi zweimalige orale Applikation des Applikationssystems vor dem Schlafengehen und Verwendung des Applikationssystems über Nacht oder über Tag zur Bildung von kristallinen Apatitablagerungen, insbesondere mit einer Schichtdicke von teilweise mindestens größer gleich 2 µm.

Gegenstand der Erfindung ist auch die Verwendung der Vernetzerlösungen, insbesondere zur Verwendung bei der Herstellung jeweils unabhängig eines Formkörpers, mit einer Vernetzerlösung (a) umfassend eine Phosphat-Mischung, die hergestellt wird durch Mischen von (i) 0.05 bis 4 mol/l, 0.5 bis 1.5 mol/l wasserlöslichen Phosphaten oder zu wasserlöslichen Phosphat-Ionen hydrolysierbaren Phosphaten, (ii) einer entsprechenden Menge Wasser oder ein Gemisch von Wasser und einem organischen Lösemittel, (iii) optional mindestens einer Carbonsäure und/oder eines Puffersystems, (iv) 0 bis 6000 Gew.-ppm wasserlöslichen Fluorids oder einer Fluorid freisetzenden Verbindung, und/oder einer Vernetzerlösung (b) umfassend eine Calcium-Mischung, die hergestellt wird durch Mischen von (i) 0.1 bis 2 mol/l Calcium-Ionen oder Calcium-Ionen freisetzenden Verbindungen, (ii) einer entsprechenden Menge Wasser oder einem Gemisch von Wasser und einem organischen Lösemittel, (iii) optional mindestens einer Carbonsäure und/oder eines Puffersystems gemischt werden, und (a) und/oder (b) mit einer definierten Menge einer Lösung enthaltend einen di- oder polyfunktionellen Vernetzer umfassend Dialdehyde, Polyepoxide, Polyisocyanate, gemischt werden, insbesondere ist der Vernetzer Glutardialdehyd. Die Phosphat-Lösung kann durch Zugabe des Vernetzers, beispielsweise Glutardialdehyd, als Vernetzerlösung verwendet werden. Ebenso kann die Calcium-Lösung durch Zugabe des Vernetzers, beispielsweise Glutardialdehyd, als Vernetzerlösung verwendet werden.

Der Vernetzer liegt dazu in einer Vernetzerlösung vor und wird mit dem geformten Gel in Kontakt gebracht. Bevorzugt sind Lösungen von 0,005 bis 90 Gew.-% Vernetzer in Lösemittel in Bezug auf die Gesamtzusammensetzung, insbesondere in Wasser oder Wasser enthaltendem Lösemittel, bevorzugt sind 0,005 bis 5 Gew-%, besonders bevorzugt 0,1 bis 4 Gew.-%, vorteilhaft um 0,1 bis 1 Gew.-%. Bevorzugt wird als Vernetzerlösung eine wässrige Glutardialdehyd-Lösung eingesetzt. Die Vernetzerlösung wird vorzugsweise hergestellt, indem die Phosphatlösung mit dem Vernetzer versetzt wird. Die bevorzugte Einwirkzeit liegt bei 1 bis 200 Sekunden, besonders bevorzugt bei 10 bis 60 Sekunden (s). Vorzugsweise wird für etwa 20 Sekunden behandelt. Der bevorzugte pH-Wert der Vernetzungslösung liegt zwischen 4.0 und 12.0. Ein anschliessendes Abspülen mit Phosphat- bzw. Calciumlösung ist möglich. Gegenstand der Erfindung ist auch eine Vernetzerlösung umfassend eine Phosphatlösung oder eine Calciumlösung sowie einen Gehalt an Vernetzer.

Ferner umfasst das Kit vorzugsweise eine Formulierung in Form a) einer wässrigen Vorbehandlungslösung synonym zu Mundspüllösung umfassend Wasser, 0.1 bis 30 Gew.-% eines gut wasserlöslichen Calcium-Salzes, insbesondere 5 bis 15 Gew.-% in Bezug auf die Gesamtzusammensetzung, vorzugsweise Calciumlactat, Calciumchlorid, Calciumgluconat, Calciumlacto-Gluconat, ein Hydrat der Salze oder eine Mischung enthaltend zwei der Salze, optional einen Gehalt eines Puffersystems, optional Maskierungsmittel oder Aromatisierungsmittel und aufweisend einen pH-Wert von 5.0 bis 12.0, oder die Formulierung in Form b) umfasst mindestens ein gut wasserlösliches Calcium-Salz, umfassend vorzugsweise Calciumlactat, Calciumchlorid, Calciumgluconat, Calciumlacto-Gluconat, ein Hydrat der Salze oder eine Mischung enthaltend zwei der Salze, optional einen Gehalt eines Puffersystems, optional einen Gehalt an Maskierungsmittel oder Aromatisierungsmittel sowie üblichen Formulierungshilfsmitteln, wie Sprengmittel, HPMC etc., insbesondere in Form eines wasserlöslichen Granulats, wasserlöslichen Pellets, Tabletten, als Sachet, Pulver bzw. Granulat in einem Sachet oder löslichen Kapseln. Gegenstand der Erfindung ist auch die Verwendung einer einzelnen Formulierung der Form b) zusammen mit einem Kit umfassend die erfindungsgemässen Formulierungen. Die vorgenannte Mundspüllösung ist eine Vorbehandlungslösung zur Vorbehandlung einzelner oder aller Zähne oder eine Formulierung aus der durch Zugabe von Wasser eine entsprechende Lösung hergestellt werden kann.

Die Zusammensetzung des Kits ist im Folgenden beschrieben. Die Mundspülung oder Vorbehandlungslösung ist zusammengesetzt aus 0.1 Gew.-% bis 30 Gew.-% eines gut wasserlöslichen Calcium-Salzes, bevorzugt 5 Gew.-% bis 15 Gew.-% Calciumchlorid oder Calciumlactat oder Calciumgluconat oder Calciumlacto-Gluconat oder anderer ausreichend lösliche Calciumsalze. Die Lösung wird mit einem geeigneten Puffer auf einen pH-Wert zwischen 5.0 und 12.0 eingestellt, bevorzugt 8,0 bis 10,0. Zur Geschmacksverbesserung ist eine Aromatisierung oder auch eine Komplexierung von geschmacksbeeinträchtigenden Verbindungen möglich, solange die Apatitabscheidung nicht beeinträchtig wird. Als Puffer eignen sich alle Puffer die in diesem pH-Wert-Bereich gute Pufferkapazität aufweisen z.B. EDTA, Tris, HEPES oder Barbital-Acetat-Puffer aber auch andere Puffersysteme, bevorzug ist Tris.

Weiter bevorzugt ist es, wenn die Formulierungen oder Mundspüllösungen mindestens ein Puffersystem, vorzugsweise eine Puffersubstanz aus der Gruppe der primären Alkalicitrate, der sekundären Alkalicitrate und/oder einem Salz von Carbonsäuren, insbesondere mit 1 bis 20 C-Atomen, vorzugsweise ein Salz mindestens einer Fruchtsäure, ein Salz einer alpha-Hydroxysäure und/oder ein Salz einer Fettsäure, insbesondere mit 1 bis 20 C-Atomen. Besonders bevorzugte Salze der vorgenannten Säuren sind die Alkali-, Erdalkali- und/oder Zink-Salze der Citronensäure, Aepfelsäure, Weinsäure und/oder Milchsäure oder Tris. Besonders bevorzugte Salze umfassen die Kationen von Natrium, Kalium, Magnesium und/oder Zink der vorgenannten Carboxylate.

Die erfindungsgemässen Formulierungen können vorzugsweise zur Behandlung empfindlicher Zähne, empfindlicher Zahnhälse, säureerodierter Zähne, rissiger Zähne, oberflächliche abradierter Zähne, freiliegender Zahnhälse, gebleichter Zähne, Zähnen nach einer Behandlung kariöser Zahnbereiche ein- (once-a-day) bis zweifach (bspw. one-day-mineralisation) angewendet werden, um eine 2 bis grösser gleich 5 µm dicke, vorzugsweise homogene und im Wesentlichen kristalline Apatitschicht auf den behandelten Oberflächen auszubilden. Grundsätzlich kann die Formulierung bei Bedarf häufiger angewendet werden, beispielsweise nach definierten Zeiträumen.

Die Erfindung wird anhand der nachfolgenden Beispiele und Figuren näher erläutert, ohne sie auf diese Beispiele zu beschränken.

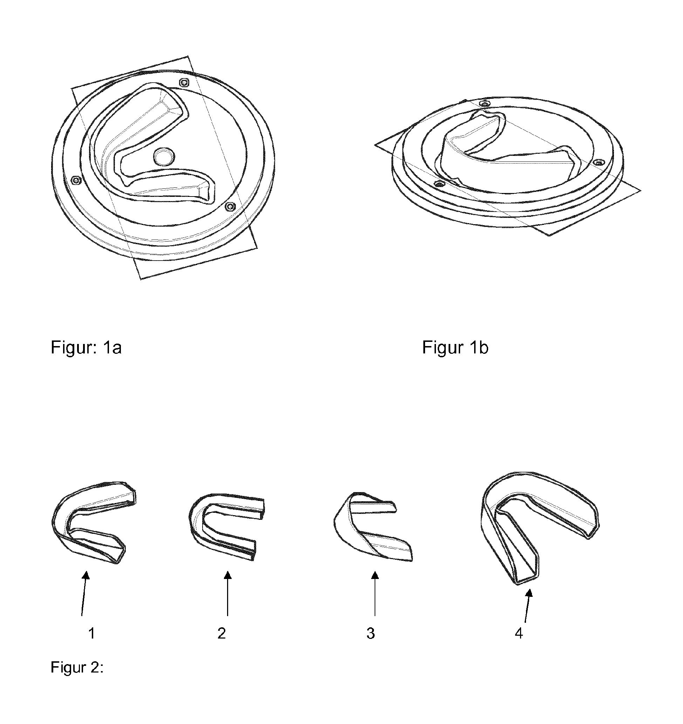

Beispiel 1: Für ein Remineralisations-Kit werden Vorbehandlungslösung, Phosphat-Komponente und Calcium-Komponente benötigt. Die Herstellung der Vorbehandlungslösung ist nachfolgend beschrieben. Für die phosphationenhaltige Komponente A wird eine Lösung hergestellt, die 29,5 g NaH₂PO₄, 33 g Olaflur, und 27,0 g Milchsäure enthält. Der pH-Wert wird mit 5 N Natronlauge auf 5,4 eingestellt und die Lösung wird mit entionisiertem Wasser auf 250 ml aufgefüllt. 24 ml der Lösung werden mit 6 g Glycerin und 10 g einer 300 Bloom Schweineschwarte-Gelatine unter Erwärmen zu einem dickflüsigen Gel (formbare Matrix) verarbeitet. Das Gel wird großzügig in das in Figur. 1a, 1b gezeigte Werkzeug eingebracht und unter 0.5 bar Druck gepresst. Nach Abkühlung wird das Werkzeug geöffnet und die Phosphat-Komponente (weiterer Formkörper) wird entnommen. Nach 40 s Spülen in einer 0.5%igen Glutardialdehyllösung reduziert sich die Löslichkeit des Gels so weit, dass es bei Mundmillieu für bis zu 8 Stunden nicht verflüssigt.

Für die Calciumionenhaltige Komponente B wird eine Calziumchloridlösung angesetzt, die 29,4 g Calciumchlorid-Dihydrat und 6,3 g Milchsäure enthält. Mit 5 N Natronlauge wird ein pH-Wert von 5.7 eingestellt. Die Lösung wird mit entionisiertem Wasser auf 200 ml aufgefüllt. Zur Herstellung des Gels werden 21.6 g der Lösung mit 8,24 g Glycerin und 8 g Calciumsulfat und 13,6 g 300 Bloom-Gelatine vermischt und erwärmt (formbare Matrix) und in die Negativ-Form des Kiefermodells (Figur. 1a, 1b) eingebracht. Anschließend wird das Werkzeug unter Druck mit der anderen Seite verschlossen und für 30 min bei -18°C abgekühlt. Die Abkühlung bewirkt eine Stabilisierung der ansonsten sehr weichen Gele in der dreidimensionalen Form und reduziert die Klebrigkeit. Das Werkzeug kann nun geöffnet werden und der erste Formkörper umfassend die Ca-Matrix entnommen werden. Nach 40 s Spülen in einer 0.5%igen Glutardialdehyllösung reduziert sich die Löslichkeit des Gels so weit, dass es bei Mundmillieu für bis zu 8 Stunden nicht verflüssigt.

Figur 1a, 1b: CAD-Modell des Werkzeugs zur Herstellung der zwei Mineralisationskomponenten in Form einer Schiene (erster oder weiterer Formkörper).

Figur 2: Die drei Komponenten: 1. Schutzschiene (Applikations-Zahnschiene), 2. Calcium-Komponente (erster Formkörper), 3. Phosphat-Komponente (weiterer Formkörper). 1 und 2 kann zu einer Form (Applikations-Zahnschiene und der erste Formkörper bilden eine Einheit) kombiniert werden, 3 (weiterer Formkörper) darf erst kurz vor Beginn der Mineralisation zugefügt werden.

Beispiel 2: Die Herstellung der Gelschienen entspricht der Beschreibung in Beispiel 1, jedoch wird die Calcium-Komponente gemeinsam mit einer thermoverformbaren Schiene hergestellt und im Anschluss gemeinsam mit dieser Schiene entnommen. Damit reduziert sich das System um eine Komponente.

Figur 2: Die Calcium-Komponente (4) wird so in Form gebracht, dass sie bereits in einer Schiene integriert ist, d.h. Applikations-Zahnschiene und der erste Formkörper bilden eine Einheit.

Für die calciumionenhaltige Komponente B wird eine Calciumchloridlösung angesetzt, die 29,4 g Calciumchlorid-Dihydrat und 6,3 g Milchsäure enthält. Mit 5 N Natronlauge wird ein pH-Wert von 4.0 eingestellt. Die Lösung wird mit entionisiertem Wasser auf 200 ml aufgefüllt. Für die Ca-Komponente wird eine Vernetzerlösung hergestellt. Dazu werden 2 g 25 Gew.-%ige Glutardialdehydlösung (GDA) mit der zuvor hergestellten Ca-Lösung auf 100 g aufgefüllt. Zur Vernetzung werden die Formkörper für 40 s der Vernetzerlösung ausgesetzt. Im Anschluss werden sie mit der Ca-Lösung ohne Vernetzerkomponente abgewaschen und trocken geblasen. Für die Vorbehandlungslösung (Mundspüllösung) wird eine 1 molare Calciumchloridlösung mit 0.1 mol Tris-Puffer versetzt und auf den pH-Wert von 9,0 eingestellt.

Vernetzunglösung Phosphat: Zur Herstellung der P-Komponente werden für die P-Lösung 5,9 g Na₂HPO₄, 9,1g Milchsäure, 6,6 g Olaflur und 0.6 g 5 M NaOH auf 50 ml mit entionisiertem Wasser aufgefüllt. Anschließend wird eine 0.375%ige Glutardialdehyd GDA-Lösung hergestellt. die Behandlung des weiteren Formkörper kann für ca. 30 sec. erfolgen.

Beispiel 3 (Abscheidung Fluorapatit): Für die phosphationenhaltige Komponente A wird eine Lösung hergestellt, die 29,5 g NaH₂PO₄, 33 g Olaflur, und 27,0 g Milchsäure enthält. Der pH-Wert wird mit 5 N Natronlauge auf 5,4 eingestellt und die Lösung wird mit entionisiertem Wasser auf 250 ml aufgefüllt. 24 ml der Lösung werden mit 6 g Glycerin und 10 g einer 300 Bloom Schweineschwarte-Gelatine unter Erwärmen zu einer dickflüssigen Lösung verarbeitet. In eine Schablone mit der Wandstärke von 500 µm wird etwas Flüssigkeit eingebracht und unter 2 bar Druck gepresst. Nach Verfestigung werden die Streifen der Schablone entnommen und in 1x1 cm grosse Quadrate geschnitten.

Für die calciumionenhaltige Komponente B wird eine Calciumchloridlösung angesetzt, die 29,4 g Calciumchlorid-Dihydrat und 6,3 g Milchsäure enthält. Mit 5 N Natronlauge wird ein pH-Wert von 4.0 eingestellt. Die Lösung wird mit entionisiertem Wasser auf 200 ml aufgefüllt. Zur Herstellung des Gels werden 21,6 g der Lösung mit 8,24 g Glycerin und 8 g Calciumsulfat und 13,6 g 300 Bloom-Gelatine vermischt und erwärmt. Das flüssige Gel wird mit einer Rakel auf eine Dicke von 1 mm ausgestrichen oder in einer Schablone mit der Wandstärke von 1 mm gepresst. Nach Verfestigung werden die Streifen werden sie 1x1 cm grosse Quadrate geschnitten.

Vorbehandlungslösung (Mundspüllösung). Es wird eine 1 molare Calciumchloridlösung mit 0.1 mol Tris-Puffer versetzt und auf den pH-Wert von 9,0 eingestellt.

Zur Bewertung der Mineralisationsaktivität werden je 6 Zahnscheiben für 10 s mit 1 M HCl angeäzt, mit der Vorbehandlungslösung abgespült und mit je einem Stück Phosphat-Gel und einem Stück Calciumgel bedeckt. Um die morphologische Änderung der Zahnoberfläche deutlich zu machen, wurde eine Scheibe zuvor zur Hälfte abgeklebt, so dass sie nur hälftig remineralisieren kann. Die Proben werden im Klimaschrank bei 37°C und 95% Luftfeuchtigkeit aufbewahrt und nach 8 bis 16 Stunden mit lauwarmen Wasser und einer weichen Zahnbürste gereinigt. Nach nur einer Behandlung ist die Zahnoberfläche zum grössten Teil mit einer fest haftenden Schicht überzogen. Die Schicht kann bis zu 2.5 µm dick sein. Die Gele waren nach 8 bis 16 Stunden auf den Proben verlaufen.

Beispiel 4a: Zur Herstellung der P-Komponente werden für die P-Lösung 5,9 g Na₂HPO₄, 9,1g Milchsäure, 6,6 g Olaflur und 0.6 g 5 M NaOH auf 50 ml mit entionisiertem Wasser aufgefüllt. Die Herstellung des Gels erfolgt wie unter Beispiel 1 beschrieben. Die Calciumlösung für die Ca-Komponente wird durch Lösen in entionisiertem Wasser von 14,7 g CaCl₂, 3,15 g Milchsäure, 10 g 5 m Natronlauge in insgesamt 100 ml Lösung hergestellt. Die Herstellung des Gels entspricht der Beschreibung in Beispiel 1. Das Gleiche gilt für die Vorbehandlungslösung.
Zur Bewertung der Mineralisationsaktivität werden je 6 Zahnscheiben für 10 s mit 1 M HCl angeäzt, mit der Vorbehandlungslösung abgespült und mit je einem Stück Phosphat-Gel und einem Stück Calziumgel bedeckt. Um die morphologische Änderung der Zahnoberfläche deutlich zu machen, wurde eine Scheibe zuvor zur Hälfte abgeklebt, so dass sie nur hälftig remineralisieren kann. Die Proben werden im Klimaschrank bei 37°C und 95 % Luftfeuchtigkeit aufbewahrt und nach 8 bis 12 Stunden mit lauwarmen Wasser und einer weichen Zahnbürste gereinigt. Nach nur einer Behandlung ist die Zahnoberfläche zum grössten Teil mit einer fest haftenden Schicht überzogen. Um die Bereiche zwischen erfolgreicher Beschichtung und freiliegendem Dentin mit dem blossen Auge sichtbar zu machen, werden die Proben nach der Reinigung mit 0.1%iger Rhodaminlösung getränkt und kurz abgespült. Mittels Farbmessgerät wird der Farbunterschied delta E zwischen der beschichteten und der unbeschichteten Seite bestimmt. Der Durchschnitttswert liegt bei 52 (STAB 12).

Beispiel 4b: Die Herstellung der Komponenten des Kits entspricht Beispiel 4a, jedoch wird das P-Gel für 30 s mit einer 0.375%igen GDA-Lösung behandelt, die durch Mischen der P-Lösung mit der entsprechenden Menge GDA hergestellt wird. Die Gelstreifen werden im Anschluss nur trocken getupft. Die Ca-Gele werden mit einer 0.25%igen GDA-Lösung 30s lang behandelt und im Anschluss trocken getupft. Die Bewertung der Mineralisationsaktivität entspricht dem Beispiel 4a. Der Durchschnittswert für delta E liegt bei 63 (STAB 5, Fehlerangabe).

Beispiel 5: Die Herstellung der Gele entspricht dem Beispiel 4b. Die Vernetzung wird aber bei beiden Gelen für jeweils 2x20s von beiden Seiten durchgeführt. Dabei liegt die GDA-Konzentration in der Ca-Vernetzerlösung bei 0.5 %. Die Bewertung der Mineralisationsaktivität entspricht den zuvor ausgeführten Beispielen. Im Elektronenmikroskop ist eine weitgehend homogene Schicht aus kleinen nadeligen Kristallen zu erkennen.

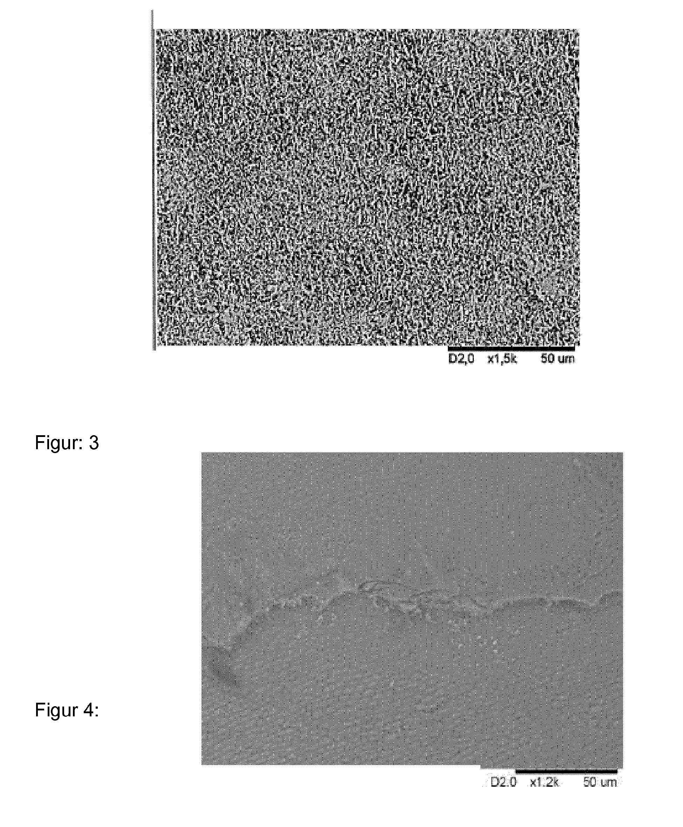

Abb. 3: Bewachsene Dentinoberfläche. Die porenreiche Dentinfläche ist von einer gleichmässigen Schicht nadeliger Kristalle überwachsen. Nach Behandlung mit dem Mineralisations-Kit (doppelseitige GDA-Vernetzung).

## Patentansprüche

1. Formkörper, **dadurch gekennzeichnet, dass** er ein teilelastischer, bogenförmiger und dreidimensionaler Formkörper ist, wobei der Formkörper eine Matrix mit einem Gel enthält und ausgewählt ist aus
a) einem ersten dreidimensionalen Formkörper enthaltend eine Matrix umfassend mindestens ein Gel und
b) einem weiteren dreidimensionalen Formkörper enthaltend eine zweite Matrix umfassend mindestens ein Gel,
und mindestens eine Matrix jeweils unabhängig mindestens einen Wirkstoff, ein pharmakologisch verträgliches Salz, Solvat eines Wirkstoffs oder ein mineralisierend wirkendes System enthält.

2. Formkörper nach Anspruch 1, **dadurch gekennzeichnet, dass** der Formkörper bogenförmig ist und eine Nut aufweist, die ausgebildet ist mindesten einen Zahn oder mindestens einen Teil eines Zahnbogen aufzunehmen, der Formkörper in Form einer Zahnschiene vorliegt oder der Formkörper zumindest teilweise einem Zahnbogennegativ entspricht, der Formkörper formschlüssig oder passgenau anliegt an mindestens einem Zahn oder Zahnbogen oder zumindest teilweise einem Zahnbogennegativ entspricht und beabstandet zu dem Zahnbogen ist.

3. Formkörper nach Anspruch 1 oder 2, **dadurch gekennzeichnet, dass**
a) das Gel des weiteren Formkörpers wasserlösliche Phosphate oder zu wasserlöslichen Phosphat-Ionen hydrolysierbare Phosphate umfasst und einen pH-Wert von 2 bis 8 aufweist, und
b) das Gel des ersten Formkörpers wasserlösliche Calcium-Ionen oder Calcium-Ionen freisetzende Verbindungen umfasst und einen pH-Wert von 3.5 bis 14 aufweist und a) das Gel des ersten Formkörpers und/oder b) das Gel des weiteren Formkörpers (i) einen Gehalt an Wasser oder ein Gemisch von Wasser und einem organischen Lösemittel, und (ii) optional mindestens eine Carbonsäure und/oder ein Puffersystem enthält.

4. Formulierung nach einem der Ansprüche 1 bis 3, **dadurch gekennzeichnet, dass** b) das Gel des weiteren Formkörpers mindestens ein wasserlösliches Fluorid oder eine Fluoride freisetzende Verbindung enthält.

5. Formkörper nach einem der Ansprüche 1 bis 4, **dadurch gekennzeichnet, dass** der erste und/oder weitere Formkörper jeweils unabhängig zumindest teilweise mindestens eine an der äußeren Oberfläche angeordnete Ebene oder eine teilweise oder im Wesentlichen vollständige äußere Hülle aufweist, und die Ebene oder die Hülle eine gegenüber wässrigen Medien verminderte Löslichkeit im Vergleich zur Matrix aufweist.

6. Formkörper nach einem der Ansprüche 1 bis 5, **dadurch gekennzeichnet, dass** das mindestens eine Gel basiert auf einem organischen Gelbilder (a) umfassend denaturiertes Kollagen, Hydrokolloiden, Hydrogele, Polypeptide, Proteinhydrolysate, synthetische Polyaminosäuren, Polysacchariden, Polyacrylaten, Polyurethane, Casein, Stärkemehl, Cellulose, HPMC, Gummi Arabicum, Galactomannane, Guarkernmehl, Konjak, Xanthan, Calciumalginat, Dextran, Scleroglucan, Pektin, Carragenan (κ-, 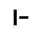 - und λ-Carrageen), Agar-Agar, Alginat, Alginsäure, Natriumalginat, Calciumalginat, Traganth oder Mischungen umfassend mindestens zwei der genannten Gelbilder,
oder anorganischem Gelbilder (b) umfassend Bentonite, Kieselgele oder Gemische enthaltend einen anorganischen Gelbilder,
(c) Silikonelastomere, oder
(d) pharmazeutische Matrixbildner, Cellulosen, Acrylate sowie auch Mischungen der Gelbilder (a), (b), (c) und/oder (d).

7. Applikationssystem zur Zahnbehandlung von Wirbeltieren, insbesondere von Menschen, umfassend mindestens eine Applikations-Zahnschiene, mit mindestens einem Formkörper nach einem der Ansprüche 1 bis 6, **dadurch gekennzeichnet, dass** die Applikations-Zahnschiene geeignet ist, mindestens einen an die Schiene angepassten ersten dreidimensionalen Formkörper enthaltend eine Matrix aufzunehmen, wobei in den ersten Formkörper mindestens ein weiterer dreidimensionaler Formkörper einsetzbar ist, wobei der weitere Formkörper eine zweite Matrix enthält.

8. Applikationssystem nach Anspruch 7, **dadurch gekennzeichnet, dass** in einer Alternative
(i) a) die Applikations-Zahnschiene einen ersten dreidimensionale Formkörper enthaltend eine Matrix aufnimmt und b) der erste dreidimensionale Formkörper einen weiteren Formkörper aufnimmt enthaltend eine Matrix, optional getrennt durch eine Membranschicht,
(ii) a) die Applikations-Zahnschiene und der erste Formkörper eine Einheit bilden und b) der erste dreidimensionale Formkörper einen weiteren Formkörper enthaltend eine Matrix aufnimmt, optional getrennt durch eine Membranschicht, oder
(iii) a) die Applikations-Zahnschiene eine Matrix umfasst und einen ersten dreidimensionalen Formkörper enthaltend eine Matrix aufnimmt; optional getrennt durch eine Membranschicht.

9. Applikationssystem nach einem der Ansprüche 7 bis 8 **dadurch gekennzeichnet, dass** (i) der weitere Formkörper eine Matrix enthält und der Formkörper zumindest teilelastisch ist, wobei die (a) Matrix umfasst
(a1) mindestens eine Mineralisationsmatrix umfassend mindestens ein Gel, umfassend
(a2) mindestens ein wasserlösliches Phosphat oder zu wasserlöslichen Phosphat-Ionen hydrolysierbare Phosphate, und
(a3) optional mindestens eine Carbonsäure und/oder ein Puffersystem
(a4) optional wasserlösliche Fluoride oder eine Fluoride freisetzende Verbindung
(a5) optional einen Gehalt an Wasser oder ein Gemisch von Wasser und einem organischen Lösemittel,
(ii) der erste Formkörper eine Matrix enthält, und der Formkörper zumindest teilelastisch ist, wobei die (b) Matrix umfasst
(b1) mindestens eine Mineralisationsmatrix umfassend mindestens ein Gel, umfassend,
(b2) wasserlösliche Calcium-Ionen oder Calcium-Ionen freisetzende Verbindungen, und
(b3) optional mindestens eine Carbonsäure und/oder ein Puffersystem
(b4) optional Wasser oder ein Gemisch von Wasser und einem organischen Lösemittel, oder
der erste Formkörper umfasst Matrices und ist zumindest teilelastisch, wobei die (c) Matrices umfassen
(c) teilelastische Formkörper C umfasst
(c1) mindestens eine Mineralisationsmatrix umfassend mindestens ein Gel, enthaltend
(c1.1) mindestens ein wasserlösliches Phosphat oder zu wasserlöslichen Phosphat-Ionen hydrolysierbares Phosphat, und
(c1.2) optional mindestens eine Carbonsäure und/oder ein Puffersystem
(c1.3) optional wasserlösliches Fluoride oder eine Fluoride freisetzende Verbindung
(c1.4) optional einen Gehalt an Wasser oder ein Gemisch von Wasser und einem organischen Lösemittel
(c2) optional eine Membran(schicht), und
(c3) mindestens eine Mineralisationsmatrix umfassend mindestens ein Gel, enthaltend
(c3.1) wasserlösliche Calcium-Ionen oder Calcium-Ionen freisetzende Verbindungen, und
(c3.2) optional mindestens eine Carbonsäure und/oder ein Puffersystem
(c3.3) optional Wasser oder ein Gemisch von Wasser und einem organischen Lösemittel, wobei der Formkörper den Schichtaufbau c1 und c3 oder c1, c2 und c3 aufweist, wobei c3 an der Applikations-Zahnschiene anliegt und c1 innen angeordnet ist, insbesondere um bei einer dentalen Anwendung an einem Wirbeltier mit mindestens einem Zahn oder einem Teil eines Zahnbogens in Kontakt zu treten.

10. Applikationssystem nach einem der Ansprüche 7 bis 9, **dadurch gekennzeichnet, dass** in einer Alternative (i) die Applikations-Zahnschiene einen ersten dreidimensionale Formkörper enthaltend eine Matrix aufweist und der erste Formkörper eine Einheit mit der Applikations-Zahnschiene bildet, wobei (b) die Matrix umfasst
(b1) mindestens eine Mineralisationsmatrix umfassend mindestens ein Gel,
(b2) wasserlösliche Calcium-Ionen oder Calcium-Ionen freisetzende Verbindungen, und
(b3) optional mindestens eine Carbonsäure und/oder ein Puffersystem
(b4) optional Wasser oder ein Gemisch von Wasser und einem organischen Lösemittel, ist oder
(ii) der erste Formkörper (b) umfassend die Mineralisationsmatrix (b1) mit (b2), (b3) und optional (b4) in die Applikations-Zahnschiene einsetzbar ist.

11. Applikationssystem nach einem der Ansprüche 7 bis 10, **dadurch gekennzeichnet, dass** in einer Alternative (i) die Applikations-Zahnschiene einen ersten Formkörper umfasst in den ein weiterer Formkörper enthaltend eine Matrix einsetzbar ist, wobei der weitere Formkörper dreidimensional ist, und die (a) Matrix umfasst
(a1) mindestens eine Mineralisationsmatrix umfassend mindestens ein Gel, umfassend
(a2) mindestens ein wasserlösliches Phosphat oder zu wasserlöslichen Phosphat-Ionen hydrolysierbare Phosphate, und
(a3) optional mindestens eine Carbonsäure und/oder ein Puffersystem
(a4) optional wasserlösliches Fluoride oder eine Fluoride freisetzende Verbindung
(a5) optional einen Gehalt an Wasser oder ein Gemisch von Wasser und einem organischen Lösemittel, oder
(ii) der weitere Formkörper (a) umfassend die Mineralisationsmatrix (a1) mit (a2), (a3), optional (4a) und optional (a5) optional getrennt durch eine Membran als Einheit mit der Applikations-Zahnschiene nach Anspruch 10 Alternative (i) vorliegt.

12. Verfahren zur Herstellung des Formkörpers nach einem der Ansprüche 1 bis 6, indem eine formbare Matrix in ein erstes Werkstück mit einer Ausnehmung, die zumindest einem teilweisen Kiefernegativ oder teilweisen Zahnbogennegativ entspricht oder davon abgeleitet ist, eingebracht wird und mit einem weiteren Werkstück, das zumindest teilweise einem Kieferpositiv oder teilweisen Zahnbogenpositiv entspricht, und unter Normaldruck oder Überdruck geformt wird, optional abgekühlt wird, nach Formung wird die geformte Matrix als Formkörper entnommen, wobei der erste oder der weitere Formkörper erhalten wird.

13. Verfahren zur Herstellung des Applikationssystem nach einem der Ansprüche 7 bis 11, indem (a) eine Applikations-Zahnschiene (a.1) vorgelegt oder
(a.2) geformt wird, insbesondere mittels Spritzguss, Extrusion oder anderen dem Fachmann bekannte Verfahren,
(b.1) der erster Formkörper in die Applikations-Zahnschiene (b.1.1) vorgeformt eingebracht wird, oder
(b.1.2) mittels 2K-Spritzguss in die Applikations-Zahnschiene eingebracht und geformt wird oder
(b.1.3) der erste Formkörper vom Anwender geformt und in die Applikations-Zahnschiene eingebracht wird.

14. Verfahren nach Anspruch 13, **dadurch gekennzeichnet**, das in dem Verfahren
(c.1) der weiterer Formkörper in die Applikations-Zahnschiene (c.1.1) vorgeformt eingebracht wird, oder
(c.1.2) mittels 2K-Spritzguss optional getrennt durch eine Membran, in den ersten Formkörper eingebracht wird, oder
(c.1.3) der weitere Formkörper vom Anwender geformt und in die den ersten Formkörper in der Applikations-Zahnschiene eingebracht wird.

15. Verfahren nach einem der Ansprüche 12 bis 14, **dadurch gekennzeichnet, dass**
a) der weitere Formkörper umfassend eine Matrix und mindestens ein Gel enthaltend mindestens ein wasserlösliches Phosphat oder Phosphat freisetzende Verbindung, Gelatine und Glycerin, optional mindestens eine Carbonsäure und/oder ein Puffersystem, optional wasserlösliche Fluoride oder eine Fluoride freisetzende Verbindung, optional einen Gehalt an Wasser oder ein Gemisch von Wasser und ein organisches Lösemittel hergestellt wird, indem,
- als formbare Matrix warmes nicht verfestigtes Gel in das erste Werkzeug eingebracht wird, unter Druck gepresst wird, optional wird abgekühlt, das verfestigte Gel wird als Matrix oder als Formkörper umfassend die Matrix entnommen, nachfolgend wird die Matrix an mindestens einer äußeren Ebene chemisch vernetzt oder außen zu einer Hülle chemisch vernetzt oder beschichtet und/oder
b) der erste Formkörper umfassend eine Matrix und mindestens ein Gel enthaltend wasserlösliche Calcium-Ionen oder Calcium-Ionen freisetzende Verbindungen, optional mindestens eine Carbonsäure und/oder ein Puffersystem, optional Wasser oder ein Gemisch von Wasser und einem organischen Lösemittel hergestellt wird, indem
- als formbare Matrix warmes nicht verfestigtes Gel in das zweite Werkzeug eingebracht wird, unter Druck gepresst wird, optional wird abgekühlt, das verfestigte Gel wird als Matrix oder als Formkörper entnommen, nachfolgend wird die Matrix an mindestens einer äußeren Ebene chemisch vernetzt oder außen zu einer Hülle chemisch vernetzt oder in einer Ebene oder mit einer Hülle beschichtet.

16. Kit umfassend ein Applikationssytem umfassend eine Applikations-Zahnschiene, einen ersten Formkörper und/oder einen weiteren Formkörper nach einem der Ansprüche 1 bis 15 sowie optional eine Vorspüllösung.

17. Verwendung eines Applikationssystems nach einen der Ansprüche 1 bis 15 oder Kit nach Anspruch 16 enthaltend Phosphat oder zu wasserlöslichen PhosphatIonen hydrolysierbare Phosphate, wasserlösliches Fluoride oder eine Fluoride freisetzende Verbindung und wasserlösliche Calcium-Ionen oder Calcium-Ionen freisetzende Verbindungen für eine remineralisierende Behandlung von Zähnen durch ein- oder mehrmalige orale Applikation des Applikationssystems vor dem Schlafengehen und Verwendung des Applikationssystems über Nacht oder über Tag zur Bildung von kristallinen Apatitablagerungen, insbesondere mit einer Schichtdicke von teilweise mindestens größer gleich 2 µm.
